(19) Europäisches Patentamt
European Patent Office
Office européen des brevets

(11) Veröffentlichungsnummer: **0 191 390**
**B1**

(12) # EUROPÄISCHE PATENTSCHRIFT

(45) Veröffentlichungstag der Patentschrift: **23.08.89**

(51) Int. Cl.⁴: **C 07 D 405/14**, C 07 D 405/12, C 07 D 409/14, A 61 K 31/495

(21) Anmeldenummer: **86101348.0**

(22) Anmeldetag: **03.02.86**

(54) **1-Cyclopropyl-1,4-dihydro-4-oxo-7- 4-(2-oxo-1,3-dioxol-4-yl-methyl)-1-piperazinyl -3-chinolincarbonsäuren, Verfahren zu ihrer Herstellung sowie diese enthaltende antibakterielle Mittel.**

(30) Priorität: **12.02.85 DE 3504643**

(43) Veröffentlichungstag der Anmeldung: **20.08.86 Patentblatt 86/34**

(45) Bekanntmachung des Hinweises auf die Patenterteilung: **23.08.89 Patentblatt 89/34**

(84) Benannte Vertragsstaaten: **AT BE CH DE FR GB IT LI NL SE**

(56) Entgegenhaltungen:
**EP-A-0 067 411**
**EP-A-0 113 091**
**EP-A-0 117 474**
**EP-A-0 126 355**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Petersen, Uwe, Dr., Auf dem Forst 4, D-5090 Leverkusen (DE)**
Erfinder: **Grohe, Klaus, Dr., Am Wasserturm 10, D-5068 Odenthal (DE)**
Erfinder: **Zeiler, Hans- Joachim, Dr., Elsbeeker Strasse 46, D-5620 Velbert 15 (DE)**
Erfinder: **Metzger, Karl Georg, Dr., Pahlkestrasse 75, D-5600 Wuppertal (DE)**

**EP 0 191 390 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 1-Cyclopropyl-1,4-dihydro-4-oxo-7-[4-(2-oxo-1,3-dioxol-4-yl)-methyl-1-piperazinyl]-3-chinolincarbonsäuren, Verfahren zu ihrer Herstellung sowie diese enthaltende antibakterielle Mittel.

Es ist bereits bekannt geworden, daß 1-Ethyl-6-fluor-1,4-dihydro-4-oxo-7-[4-(5-methyl-2-oxo-1,3-dioxol-4-yl-methyl)-1-piperazinyl]-3-chinolincarbonsäure (EP-A-67 411) und 7-[4-(5-tert.-Butyl-2-oxo-1,3-dioxol-4-yl-methyl)-1-piperazin-yl]-1-ethyl-6-fluor-1,4-dihydro-4-oxo-3-chinolincarbonsäure (Japanische Patentanmeldung 58 069 880) und deren Säureadditionssalze als antimikrobielle Wirkstoffe verwendet werden können.

Es wurde nun gefunden, daß die neuen 1-Cyclopropyl-1,4-dihydro-4-oxo-7-[4-(2-oxo-1,3-dioxol-4-yl-methyl)-1-piperazinyl]-3-chinolincarbonsäuren der Formel (I)

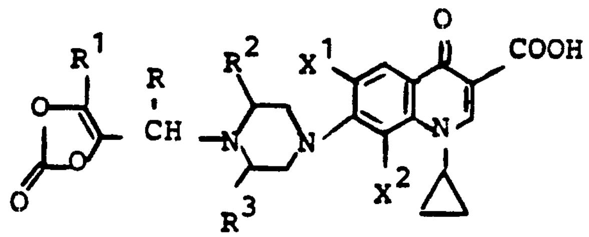

(I),

in welcher

R für Wasserstoffatom steht oder mit $R^1$ gemeinsam einen Alkylenrest mit 2 oder 3 Kohlenstoffatomen bildet,
$R^1$ für ein Wasserstoffatom, Alkyl mit 1 bis 4 Kohlenstoffatomen oder Phenyl steht,
$R^2$ und $R^3$ gleich oder verschieden sein können und für ein Wasserstoffatom, Methyl, Ethyl, Cyclohexyl, Methylendioxyphenyl, Furyl, Tetrahydrofuryl, Thienyl oder für gegebenenfalls durch Fluor, Chlor, Brom, Methyl, Phenyl, Cyano, Hydroxy, Methoxy, Benzyloxy, Amino, Methylamino, Dimethylamino, Piperidino oder Nitro ein- bis dreifach substituiertes Phenyl stehen,
$X^1$ für ein Wasserstoff-, Fluor-, Chlor- oder Bromatom, oder Nitro und
$X^2$ für ein Wasserstoff-, Fluor-, Chlor- oder Bromatom

stehen, und deren pharmazeutisch verwendbaren Hydrate, Säureadditionssalze, Alkali- und Erdalkalisalze eine hohe antibakterielle Wirkung aufweisen.

Bevorzugt sind diejenigen Verbindungen der Formel (I), in denen

R für ein Wasserstoffatom steht,
$R^1$ für Methyl oder tert.-Butyl steht,
$R^2$ und $R^3$ gleich oder verschieden sein können und für Wasserstoffatom, Methyl, Ethyl, Cyclohexyl, Thienyl oder für gegebenenfalls durch Fluor, Chlor, Brom, Methyl, Phenyl, Cyano, Hydroxy, Methoxy, Amino, Piperidino oder Nitro ein- bis dreifach substituiertes Phenyl stehen,
$X^1$ für Wasserstoff-, Fluor-, Chlor- oder Bromatom, oder Nitro und
$X^2$ für Wasserstoff-, Fluor-, Chlor- oder Bromatom steht.

Besonders bevorzugt sind diejenigen Verbindungen der Formel (I), in denen

R für ein Wasserstoffatom steht,
$R^1$ für Methyl steht,
$R^2$ und $R^3$ gleich oder verschieden sein können und für ein Wasserstoffatom, Methyl, Cyclohexyl, Thienyl oder für gegebenenfalls durch Fluor, Chlor, Brom, Methyl, Hydroxy, Methoxy, Benzyloxy, Amino, Piperidino oder Nitro ein- oder zweifach substituiertes Phenyl stehen,
$X^1$ für ein Fluor-, Chlor- oder Bromatom und
$X^2$ für ein Wasserstoff- Fluor-, Chlor- oder Bromatom steht.

Weiterhin wurde gefunden, daß man die Verbindungen der Formel (I) erhält, wenn man 1-Cyclopropyl-1,4-dihydro-4-oxo-7-(1-piperazinyl)-3-chinolincarbonsäuren der Formel (II)

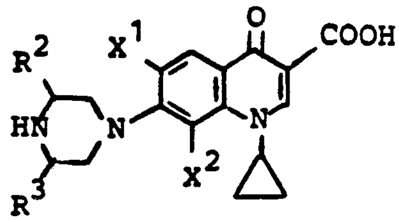

(II),

in welcher $R^2$, $R^3$, $X^1$ und $X^2$ die oben angegebene Bedeutung haben, mit Verbindungen der Formel (III)

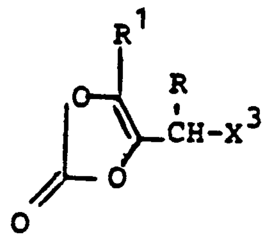

(III),

in welcher $R^1$ und R die oben angegebene Bedeutung haben und

$X^3$ für ein Halogenatom, wie beispielsweise ein Chlor-, Brom- oder Jodatom, oder einen Rest $O\text{-}SO_2\text{-}R^4$ steht, wobei

$R^4$ Methyl, Trifluormethyl, Phenyl oder 4-Methylphenyl bedeutet,

gegebenenfalls in Gegenwart von Säurebindern umsetzt.

Verwendet man bei der erfindungsgemäßen Umsetzung zum Beispiel 1-Cyclopropyl-6-fluor-1,4-dihydro-4-oxo-7-(1-piperazinyl)-3-chinolincarbonsäure und 4-Brommethyl-5-methyl-1,3-dioxol-2-on als Ausgangsverbindungen, so kann der Reaktionsablauf durch das folgende Formelschema wiedergegeben werden:

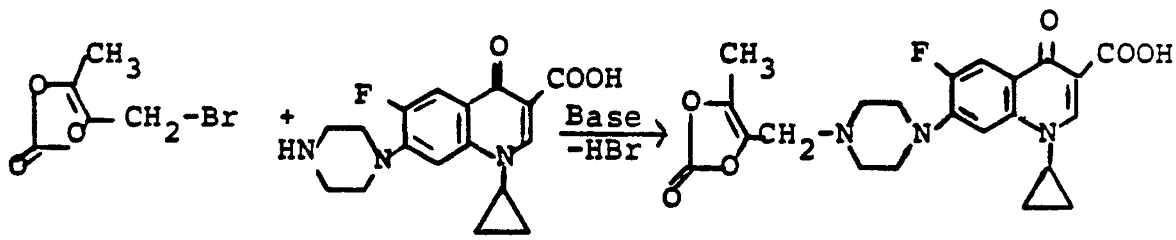


Die als Ausgangsstoffe verwendbaren halogensubstituierten 1-Cyclopropyl-1,4-dihydro-4-oxo-7-(1-piperazinyl)-3-chinolincarbonsäuren der Formel (II) sind teilweise bekannt (vergleiche DE-OS-3 142 854, DE-OS-3 248 506, DE-OS-3 308 909, DE-OS-3 318 145) oder können gemäß folgendem Formelschema hergestellt werden:

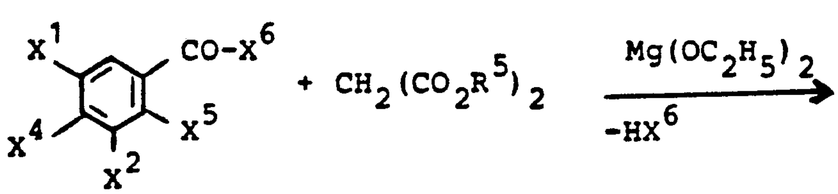


(1) (2)

$X^4$, $X^5$, $X^6$ = F oder Cl

$R^5 = CH_3, C_2H_5, C_3H_7$

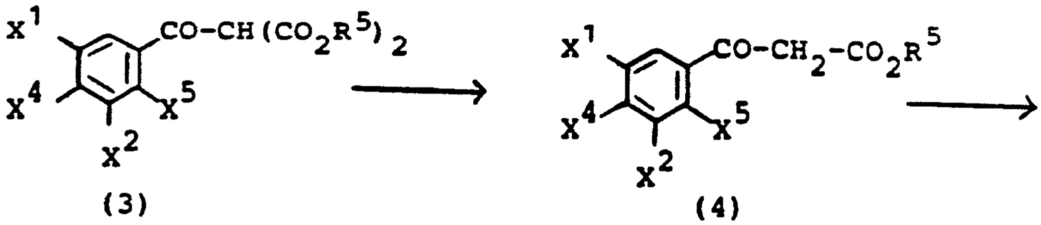

(3) (4)

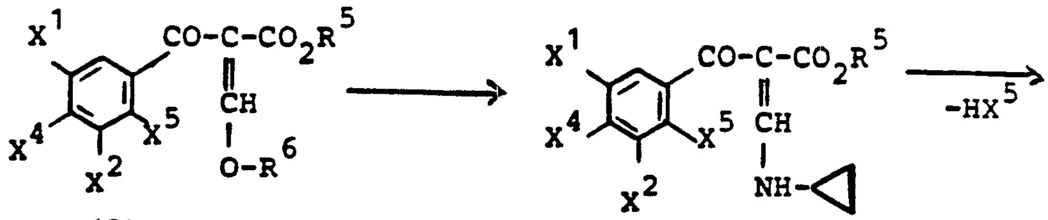


(5) (6)

$R^6 = CH_3, C_2H_5$

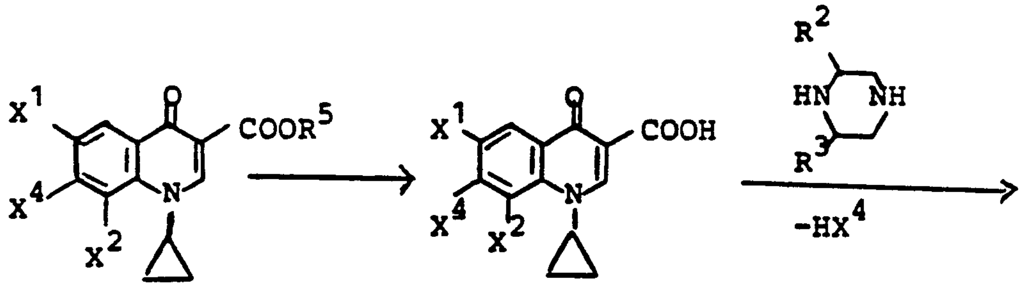


(7) (8)

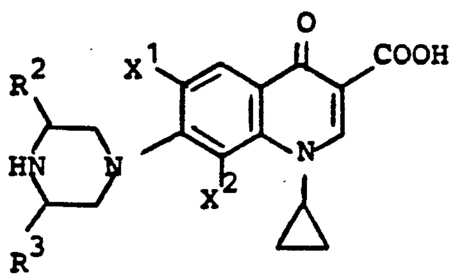

(II)

Danach wird Malonsäuredialkylester (2) in Gegenwart von Magnesiumethylat mit dem entsprechenden Benzoylfluorid bzw. -chlorid (1) zum Aroylmalonester (3) acyliert (Organicum, 3. Auflage, 1964, S. 438).

Durch partielle Verseifung und Decarboxylierung von (3) in wäßrigem Medium mit katalytischen Mengen Schwefelsäure oder p-Toluolsulfonsäure erhält man in guter Ausbeute den Aroylessigsäurealkylester (4), der mit Orthoameisensäure-trialkylester/Acetanhydrid in den entsprechenden 2-Benzoyl-3-alkoxy-acrylsäure-alkylester (5) übergeht. Die Umsetzung von (5) mit Cyclopropylamin in einem Lösungsmittel, wie z. B. Methylenchlorid, Chloroform, Methanol, Ethanol, Cyclohexan oder Toluol führt in leicht exothermer Reaktion zum gewünschten Zwischenprodukt (6).

Die Cyclisierungsreaktion (6) → (7) wird in einem Temperaturbereich von etwa 60 bis 300°C, bevorzugt 80 bis 180°C durchgeführt.

Als Verdünnungsmittel können Dioxan, Dimethylsulfoxid, N-Methylpyrrolidon, Sulfolan, Hexamethylphosphorsäuretrisamid und bevorzugt N,N-Dimethylformamid verwendet werden.

Als Säurebinder kommen für diese Reaktionsstufe Kalium-tert.-butanolat, Butyl-lithium, Lithium-phenyl, Phenylmagnesiumbromid, Natriummethylat, Natriumhydrid, Natrium- oder Kaliumcarbonat in Betracht. Wenn bei dieser Cyclisierungsreaktion Fluorwasserstoff abgespalten werden soll, verwendet man besonders bevorzugt Kalium- oder Natrium-fluorid. Es kann von Nutzen sein, einen Überschuß von 10 Mol-% an Base einzusetzen.

Die anschließende Esterhydrolyse von (7) unter basischen oder sauren Bedingungen führt zu den 1-Cyclopropyl-1,4-dihydro-4-oxo-3-chinolincarbonsäuren (8), die im letzten Schritt mit Piperazin oder substituierten Piperazinen zu den entsprechenden 1-Cyclopropyl-1,4-dihydro-4-oxo-7-(1-piperazinyl)-3-chinolincarbonsäuren (II) umgesetzt werden.

Einige der als Ausgangsstoffe für diesen Syntheseweg verwendeten Benzoylhalogenide (1) sind neu und werden wie folgt hergestellt:

3,5-Dichlor-2,4-difluor-benzoylfluorid (Siedepunkt 97°C/20 mbar; $n^{20}_D$ = 1,5148) und 5-Chlor-2,3,4-trifluorbenzoylfluorid (Siedepunkt 68 - 70°/20 mbar; $n^{20}_D$ = 1,4764) erhält man nebeneinander beim Erhitzen von Tetrachlorbenzoylchlorid mit Kaliumfluorid in Sulfolan auf erhöhte Temperaturen:

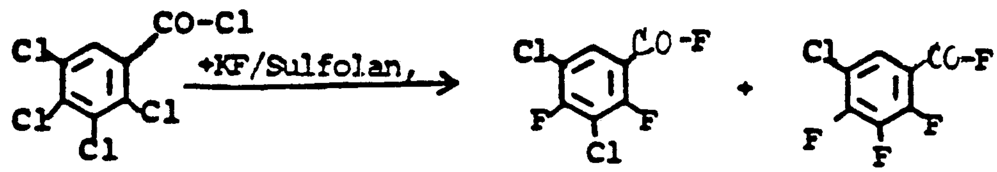


Die Chlorierung von 2,4,5-Trifluorbenzoesäure in Chlorsulfonsäure führt zur 3-Chlor-2,4,5-trifluorbenzoesäure, die als Rohprodukt mit Thionylchlorid zum 3-Chlor-2,4,5-trifluorbenzoylchlorid (Siedepunkt 94°C/18 mbar; $n^{20}_D$ = 1,5164) umgesetzt wird:

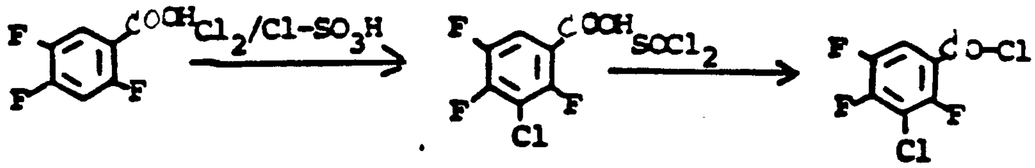


Die als Ausgangsstoffe verwendbaren reaktiven 1,3-Dioxol-2-one der Formel (III) sind aus der Literatur bekannt oder können nach literaturbekannten Verfahren erhalten werden: EP-A-39 086, 39 477, 67 411, 78 413, 90 344, US-Patentanmeldung 4 434 173, Japanische Patentanmeldungen 58 069 875 und 59 025 386. Als Beispiele seien genannt: 4-Brommethyl-1,3-dioxol-2-on, 4-Brommethyl-5-methyl-1,3-dioxol-2-on, 4-Chlormethyl-5-methyl-1,3-dioxol-2-on, 4-Methylsulfonyloxymethyl-1,3-dioxol-2-on, 4-Trifluormethylsulfonyloxymethyl-5-methyl-1,3-dioxol-2-on, 4-Brommethyl-5-tert.-butyl-1,3-dioxol-2-on, 4-Brommethyl-5-phenyl-1,3-dioxol-2-on, 3-Brom-1,2-carbonyldioxy-cyclohexen.

Die Umsetzung von II mit III wird vorzugsweise in einem Verdünnungsmittel wie Dimethylsulfoxid, N,N-Dimethylformamid, Hexamethylphosphorsäuretrisamid, Sulfolan, Diethylenglykoldimethylether oder Pyridin in Gegenwart eines säurebindenden Mittels vorgenommen. Ebenso können auch Gemische dieser Lösungsmittel verwendet werden.

Als Säurebinder können alle üblichen anorganischen und organischen Säurebindungsmittel verwendet werden.

Hierzu gehören vorzugsweise die Alkalimetallhydroxide, Alkalimetallhydrogencarbonate, Alkalimetallcarbonate, organische Amine und Amidine. Ale besonders geeignet seien im einzelnen genannt: Natriumhydroxid, Kaliumhydroxid, Natriumhydrogencarbonat, Kaliumhydrogencarbonat, Natriumcarbonat, Kaliumcarbonat, Triethylamin, 1,4-Diaza-bicyclo[2,2,2]octan (DABCO) oder 1,8-Diaza-bicyclo[5,4,0]undec-7-en (DBU).

Die Reaktionstemperaturen können in einem größeren Bereich variiert werden. Im allgemeinen arbeitet man zwischen etwa -20 und +150°C, vorzugsweise zwischen 0 und 40°C.

Die Umsetzung kann bei Normaldruck, aber auch bei erhöhtem Druck durchgeführt werden. Im allgemeinen arbeitet man bei Drucken zwischen etwa 1 und etwa 100 bar, vorzugsweise zwischen 1 und 10 bar.

Bei der Durchführung des erfindungsgemäßen Verfahrens setzt man auf 1 Mol der Carbonsäure (II) 1 bis 3 Mol, vorzugsweise 1 bis 1,5 Mol der Verbindung (III) ein.

Außer den in den Beispielen aufgeführten Verbindungen seien als neue Wirkstoffe im einzelnen genannt:

1-Cyclopropyl-6-fluor-1,4-dihydro4-oxo-7-[4-(2-oxo-1,3-dioxol-4-yl-methyl)-1-piperazinyl]-3-chinolincarbonsäure,

7-[4-(5-tert.-Butyl-2-oxo-1,3-dioxol-4-yl-methyl)-1-piperazinyl]-1-cyclopropyl-6-fluor-1,4-dihydro-4-oxo-3-chinolincarbonsäure,

1-Cyclopropyl-6-fluor-1,4-dihydro-4-oxo-7-[4-(2-oxo-5-phenyl-1,3-dioxol-4-yl-methyl)-1-piperazinyl]-3-chinolincarbonsäure,

1-Cyclopropyl-6-fluor-1,4-dihydro-4-oxo-7-[4-(4,5,6,7-tetrahydro-2-oxo-1,3-benzdioxol-4-yl)-1-piperazinyl]-3-chinolincarbonsäure,

1-Cyclopropyl-6,8-difluor-1,4-dihydro-7-[3,5-dimethyl-4-(5-methyl-2-oxo-1,3-dioxol-4-yl-methyl)-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-phenyl-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-(2-thienyl)-1-piperazinyl]-4-oxo-3-chinolincarbonsäure.

**Beispiel für eine erfindungsgemäße Tablette**

| Jede Tablette enthält: | |
|---|---|
| Verbindung des Beispiels 1 | 583,0 mg |
| Mikrokristalline Cellulose | 55,0 mg |
| Maisstärke | 72,0 mg |
| Poly-(1-vinyl-2-pyrrolidon) unlöslich | 30,0 mg |
| Hochdisperses Siliciumdioxid | 5,0 mg |
| Magnesiumstearat | 5,0 mg |
| | 750,0 mg |

| Die Lackhülle enthält: | |
|---|---|
| Poly-(0-hydroxypropyl-0-methyl)-cellulose 15 cp | 6,0 mg |
| Macrogol 4000 Rec. INN (Polyethylenglykol DAB) | 2,0 mg |
| Titan(IV)-oxid | 2,0 mg |
| | 10,0 mg |

Die erfindungsgemäßen Verbindungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z. B. Penicilline, Cephalosporine, Aminoglykoside, Sulfonamide, Tetracycline.

Diese wertvollen Eigenschaften ermöglichen ihre Verwendung als chemotherapeutische Wirkstoffe in der Medizin sowie als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Verbindungen sind gegen ein sehr breites Spektrum von Mikroorganismen wirksam. Mit ihrer Hilfe können gram-positive und gram-negative Bakterien sowie bakterienähnliche Mikroorganismen bekämpft und die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Gram-positive Kokken, z. B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z. B. Escherichia coli, Hämophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z. B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z. B. Mycobacterium tuberculosis.

Die obige Aufzählung von Erregern ist lediglich beispielhaft und keineswegs beschränkend aufzufassen. Als Krankheiten, die durch die erfindungsgemäßen Verbindungen verhindert, gebessert und/oder geheilt werden können, seien beispielsweise genannt:

Otitis; Pharyngitis; Pneumonie; Peritonitis; Pyelonephritis; Cystitis; Endocarditis; Osteomyelitis; Bronchitis; Arthritis; lokale Infektionen; septische Erkrankungen.

Zur Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur Erfindung gehören auch pharmazeutische Zubereitungen in Dosierungseinheiten. Dies bedeutet, daß die Zubereitung in Form einzelner Teile, z. B. Tabletten, Dragees, Kapseln, Pillen, Suppositorien und Ampullen vorliegt, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate,

Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Paraffin und (f) Resorptionsbeschleuniger, z. B. quartäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben aufgeführten Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rektal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis 250, insbesondere 3 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen aureichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. mit Futterzubereitungen oder mit dem Trinkwasser gegeben werden. Dadurch kann eine Infektion durch gram-negative oder gram-positive Bakterien verhindert, gebessert und/oder geheilt werden und dadurch

eine Förderung des Wachstums und eine Verbesserung der Verwertung des Futters erreicht werden.
In der nachstehenden Tabelle sind die MHK-Werte angegeben.

**Tabelle:** MHK-Werte der Verbindung aus Beispiel 1 (Agardilutionstest / Isosensitestagar)

| | |
|---|---|
| E. coli Neumann | ⩽0,015 |
| Klebsiella 8085 | ⩽0,015 |
| Proteus vulgaris 1017 | ⩽0,015 |
| Providencia 12012 | 0,03 |
| Serratia 16040 | 8 |
| Staphylococcus FK 422 | 0,25 |
| Streptococcus 27101 | 0,5 |
| Pseudomonas Ellsworth | 0,06 |

**Herstellung der Ausgangsprodukte**

**Beispiel A**

6-Chlor-1-cyclopropyl-7,8-difluor-1,4-dihydro-4-oxo-3-chinolincarbonsäure

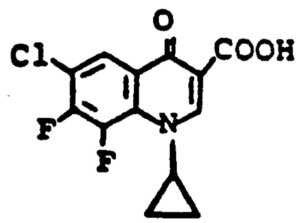

15,7 g (0,65 mol) Magnesiumspäne werden in 40 ml Ethanol und 2 ml Tetrachlormethan verrührt und nach begonnener Reaktion bei 50 - 60°C tropfenweise mit 103 g (0,64 mol) Malonsäurediethylester in 80 ml Ethanol und 250 ml Toluol versetzt. Man rührt 1 Stunde bei dieser Temperatur nach, kühlt auf -5 bis -10°C, tropft eine Lösung von 138 g (0,65 mol) 5-Chlor-2,3,4-benzoylfluorid in 63 ml Toluol zu, rührt noch 1 Stunde bei 0°C und läßt über Nacht bei Raumtemperatur stehen. Danach wird noch 2 Stunden auf 40 - 50°C erwärmt, abgekühlt und mit 250 ml Eiswasser und 38,5 ml konzentrierter Schwefelsäure versetzt. Die organische Phase wird abgetrennt, die wäßrige Phase mit 2 mal 150 ml Toluol extrahiert, die vereinigten organischen Phasen mit gesättigter Natriumchlorid-Lösung gewaschen, mit Natriumsulfat getrocknet und eingeengt.

Der Rückstand wird mit 200 ml Wasser versetzt (vorteilhaft ist hier die Zugabe von 0,4 g 4-Toluolsulfonsäure) und zur Desethoxycarbonylierung 5 Stunden unter Rückfluß erhitzt. Man extrahiert mit 3 mal 200 ml Dichlormethan, wäscht mit gesättigter Natriumchlorid-Lösung, trocknet mit Natriumsulfat, engt ein und destilliert im Hochvakuum. Man erhält 103 g (56,5 %) (5-Chlor-2,3,4-trifluor-benzoyl)-essigsäure-ethylester mit einem Siedepunkt von 110°C/0,9 Torr.

103 g (0,37 mol) des erhaltenen Esters und 83 g (0,56 mol) Orthoameisensäuretriethylester werden mit 95 g Essigsäureanhydrid 2 Stunden auf 150 - 160°C erhitzt und anschließend bei 120 - 130°C unter Normaldruck, danach im Hochvakuum eingeengt. Man erhält 115 g (92 % der Theorie) 2-(5-Chlor-2,3,4-trifluor-benzoyl)-3-ethoxyacrylsäureethylester als Öl.

84,1 g (0,25 mol) dieser Verbindung werden in 170 ml Ethanol unter Eiskühlung tropfenweise mit 14,8 g (0,26 mol) Cyclopropylamin versetzt und 2 Stunden bei Raumtemperatur gerührt. Danach wird mit 170 ml Wasser verrührt, in Eis gekühlt, der ausgefallene Niederschlag abgesaugt, mit Wasser und wenig Methanol gewaschen und getrocknet. Es werden 47 g (54 %) 2-(5-Chlor-2,3,4-trifluor-benzoyl)-3-cyclopropylamino-acrylsäureethylester vom Schmelzpunkt 71 - 73°C erhalten. Nach dem $^1$N-NMR-Spektrum liegt ein cis-trans-Gemisch vor.

47 g (0,14 mol) dieser Verbindung werden in 230 ml Dimethylformamid mit 9,7 g (0,23 mol) Natriumfluorid 2 Stunden auf 160 - 170°C erhitzt. Das Reaktionsgemisch wird in 400 ml Eiswasser eingegossen, der Niederschlag abgesaugt, mit Wasser gewaschen und getrocknet. Man isoliert 44 g (99 %) 6-Chlor-1-cyclopropyl-7,8-difluor-1,4-dihydro-4-oxo-3-chinolincarbonsäure-ethylester vom Schmelzpunkt 169 - 172°C.

44 g (0,13 mol) des Chinolincarbonsäureesters werden in 300 ml Eisessig und 179 ml Wasser mit 33 ml konzentrierter Schwefelsäure versetzt und 2 Stunden auf 150°C erhitzt. Das Reaktionsgemisch wird in 400 ml Eiswasser eingerührt, der Niederschlag abgesaugt, mit Wasser gewaschen und getrocknet. Es werden 37 g (95 % der Theorie) 6-Chlor-1-cyclopropyl-7,8-difluor-1,4-dihydro-4-oxo-3-chinolincarbonsäure mit einem Schmelzpunkt von 200 - 204°C isoliert.

**Beispiel B**

8-Chlor-1-cyclopropyl-6,7-difluor-1,4-dihydro-4-oxo-3-chinolincarbonsäure

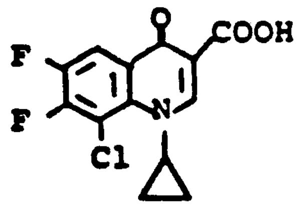

Man setzt 3-Chlor-2,4,5-trifluor-benzoylchlorid analog Beispiel A um, wobei folgende Stufen durchlaufen werden:

(3-Chlor-2,4,5-trifluor-benzoyl)-essigsäure-ethylester als Enol (Ausbeute: 42 %, Schmelzpunkt 72 - 75° C),

2-(3-Chlor-2,4,5-trifluor-benzoyl)-3-ethoxy-acrylsäure-ethylester (Rohausbeute: 95 % Öl), 2-(3-Chlor-2,4,5-trifluor-benzoyl-3-cyclopropylamino-acrylsäureethylester (Ausbeute: 67 %, Schmelzpunkt 78 - 80° C),

8-Chlor-1-cyclopropyl-6,7-difluor-1,4-dihydro-4-oxo-3-chinolincarbonsäure-ethylester (Ausbeute: 85 %, Schmelzpunkt 154 - 157° C),

8-Chlor-1-cyclopropyl-6,7-difluor-1,4-dihydro-4-oxo-3-chinolincarbonsäure (Ausbeute: 97,6 %, Schmelzpunkt 189 - 192° C).

**Beispiel C**

6,8-Dichlor-1-cyclopropyl-7-fluor-1,4-dihydro-4-oxo-3-chinolincarbonsäure

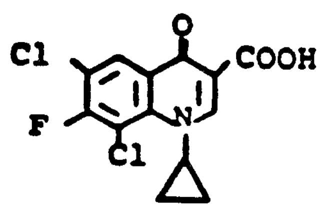

Man setzt 3,5-Dichlor-2,4-difluor-benzoylfluorid analog Beispiel A um, wobei folgende Stufen durchlaufen werden:

(3,5-Dichlor-2,4-difluor-benzoyl)-essigsäure-ethylester (Ausbeute: 43 %, Siedepunkt 133° C/2,8 Torr),

2-(3,5-Dichlor-2,4-difluor-benzoyl)-3-ethoxy-acrylsäure-ethylester (Rohausbeute: 91 % Öl),

2-(3,5-Dichlor-2,4-difluor-benzoyl)-3-cyclopropylamino-acrylsäureethylester (Ausbeute: 96 %, Schmelzpunkt 71 - 74° C),

6,8-Dichlor-1-cyclopropyl-7-fluor-1,4-dihydro-4-oxo-3-chinolincarbonsäure-ethylester (Ausbeute: 97 %, Schmelzpunkt 215 - 217° C unter Zersetzung),

6,8-Dichlor-1-cyclopropyl-7-fluor-1,4-dihydro-4-oxo-3-chinolincarbonsäure (Ausbeute: 93 %; Schmelzpunkt 204 - 206° C).

**Beispiel A1**

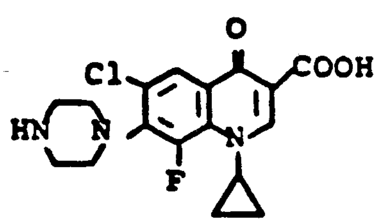

12 g (40 mmol) des Produktes aus Beispiel A werden in 100 ml Pyridin mit 17,2 g (0,2 mol) Piperazin 5 Stunden unter Rückfluß erhitzt. Das Gemisch wird im Vakuum eingeengt, mit 120 ml Wasser verrührt und mit 2 n

Salzsäure auf pH 5 eingestellt. Der Niederschlag wird abgesaugt, mit Wasser und Methanol gewaschen, in 80 ml Methanol aufgekocht und getrocknet. Man erhält 12,3 g (84 % der Theorie) 6-Chlor-1-cyclopropyl-8-fluor-1,4-dihydro-4-oxo-7-(1-piperazinyl)-3-chinolincarbonsäure vom Schmelzpunkt 295 - 298° C (unter Zersetzung).

Analog Beispiel A1 erhält man folgende in 7-Stellung substituierte 6-Chlor-1-cyclopropyl-8-fluor-1,4-dihydro-4-oxo-3-chinolincarbonsäuren:

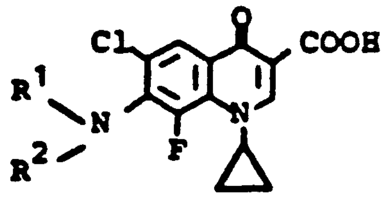

| Beispiel | 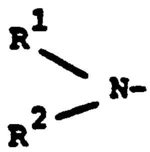 | Schmelzpunkt |
|---|---|---|
| A2 | 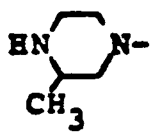 | 258 - 282° C (Zersetzung) |
| A3 | 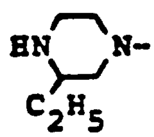 | 191 - 195° C (Zersetzung) |
| A4 | 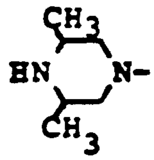 | 255 - 261° C (Zersetzung) |
| A5 | 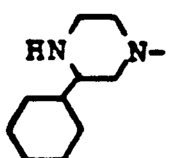 | ab ~ 190° C (Zersetzung) |
| A6 | 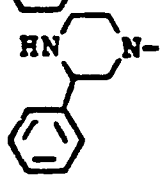 | 154 - 158° C |

**Beispiel B1**

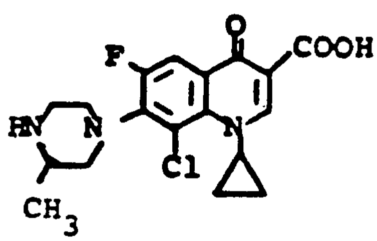

3 g (0,01 mol) des Produktes aus Beispiel B werden in 25 ml Pyridin mit 4 g (0,04 mol) 2-Methyl-piperazin 5 Stunden unter Rückfluß erhitzt. Man engt im Vakuum ein, versetzt mit 20 ml Wasser, stellt mit 2 n Salzsäure auf pH 5 und kristallisiert den ausgefallenen Niederschlag aus Methanol um. Es werden 0,6 g (16 % der Theorie) 8-Chlor-1-cyclopropyl-6-fluor-1,4-dihydro-7-(3-methyl-1-piperazinyl)-4-oxo-3-chinolincarbonsäure vom Schmelzpunkt 318 - 325° C (unter Zersetzung) erhalten.

**Beispiel B2**

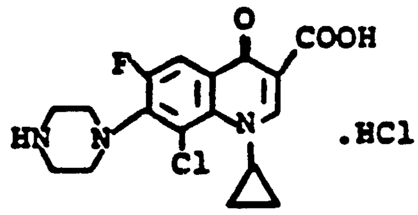

Man setzt analog Beispiel B1 das Produkt aus Beispiel B mit Piperazin 1,5 Stunden unter Rückfluß um und behandelt das Reaktionsgemisch mit Salzsäure, wobei 8-Chlor-1-cyclopropyl-6-fluor-1,4-dihydro-7-(1-piperazinyl)-3-chinolincarbonsäure-Hydrochlorid mit einem Zersetzungspunkt oberhalb 330° C erhalten wird.

**Beispiel C1**

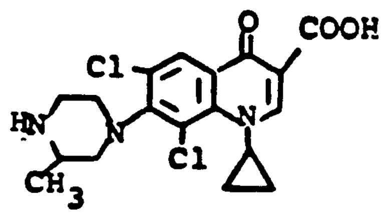

Setzt man das Produkt aus Beispiel C analog Beispiel B1 mit 2-Methyl-piperazin um, dann erhält man 6,8-Dichlor-1-cyclopropyl-1,4-dihydro-7-(3-methyl-1-piperazinyl)-4-oxo-3-chinolincarbonsäure vom Schmelzpunkt 288 - 291° C (unter Zersetzung).

**Beispiel D1**

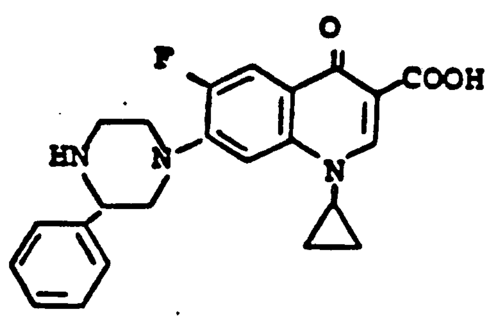

Eine Mischung aus 2,8 g (0,01 mol) 7-Chlor-1-cyclopropyl-6-fluor-1,4-dihydro-4-oxo-3-chinolincarbonsäure (DE-OS-3 142 854), 1,8 g (0,011 mol) 2-Phenyl-piperazin und 2,2 g (0,02 mol) 1,4-Diazabicyclo[2,2,2]octan in 6 ml Dimethylsulfoxid wird 4 Stunden auf 140° C erhitzt. Die Lösung wird im Hochvakuum eingeengt, der Rückstand mit 20 ml Wasser verrührt und mit 2n Salzsäure auf pH 7 eingestellt. Der Niederschlag wird abgesaugt, mit Wasser und Methanol gewaschen und in 30 ml Methanol aufgekocht. Man erhält 1,3 g (32 % der Theorie) 1-Cyclopropyl-6-fluor-1,4-dihydro-4-oxo-7-(3-phenyl-1-piperazinyl)-3-chinolincarbonsäure vom Schmelzpunkt 218 - 220° C (unter Zersetzung) (umkristallisiert aus Glykolmonomethylether).

Analog Beispiel D1 werden unter Verwendung der entsprechenden Piperazine die folgenden Verbindungen erhalten:

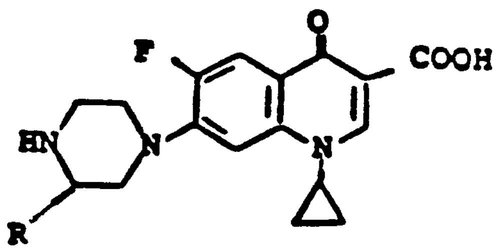

| Beispiel | R | Schmelzpunkt (unter Zersetzung) |
|---|---|---|
| D2 | 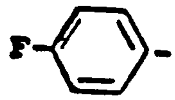 | 198 - 203° C |
| D3 | 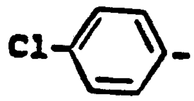 | 207 - 209° C (aus Methanol) |
| D4 | 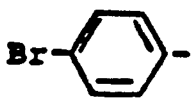 | 252 - 255° C |
| D5 | 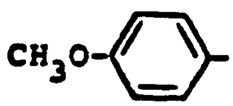 | 208 - 211° C (aus Ethanol) |
| D6 | 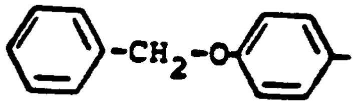 | 208 - 211° C (aus Methanol) |
| D7 | 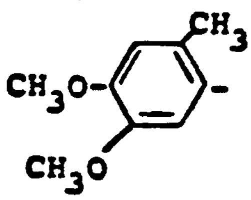 | 233 - 237° C (aus Glykolmonomethylether) |
| D8 | 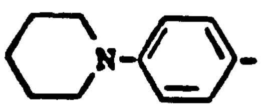 | 156 - 161° C |
| D9 | 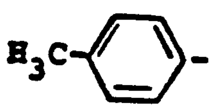 | 258 - 261° C |
| D10 | 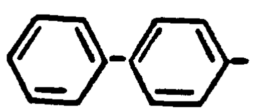 | 278 - 281° C |
| D11 | 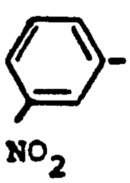 | 247 - 250° C (aus Methanol) |
| D12 | 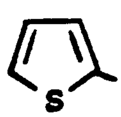 | 218 - 222° C (aus Acetonitril) |
| D13 | 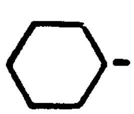 .HCl | 316 - 320° C (aus Methanol) |

**Beispiel D14**

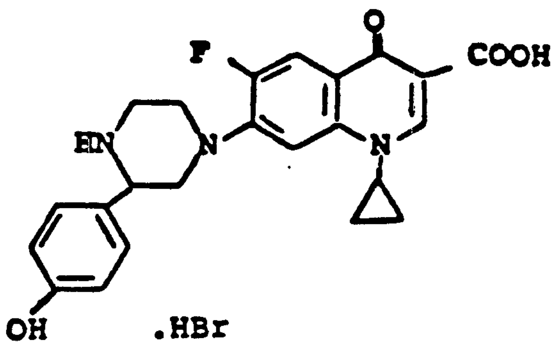

2,5 g (4,9 mmol) des Produktes aus Beispiel D6 in 30 ml Ethanol werden mit 60 ml 48 %-iger Bromwasserstoffsäure versetzt und 1 Stunde bei 55°C gerührt. Die Mischung wird im Vakuum eingeengt, der Rückstand mit 50 ml Wasser verrührt, der Niederschlag abgesaugt und mit Methanol gewaschen. Man erhält 1,5 g (66 % der Theorie) 1-Cyclopropyl-6-fluor-1,4-dihydro-7-[3-(4-hydroxy-phenyl)-1-piperazinyl]-4-oxo-3-chinolincarbonsäure-Hydrobromid vom Schmelzpunkt 295 - 298°C (unter Zersetzung).

**Herstellung der Wirkstoffe**

**Beispiel 1**

1-Cyclopropyl-6-fluor-1,4-dihydro-7-[4-(5-methyl-2-oxo-1,3-dioxol-4-yl-methyl)-1-piperazinyl]-4-oxo-3-chinolincarbonsäure

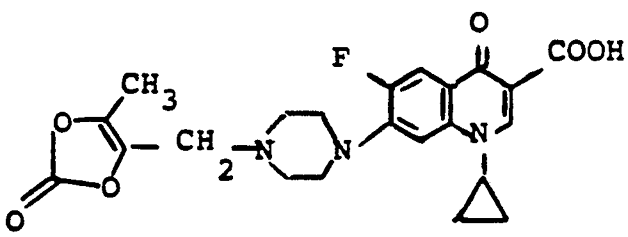

1,15 g (3,5 mmol) 1-Cyclopropyl-6-fluor-1,4-dihydro-4-oxo-7-(1-piperazinyl)-3-chinolincarbonsäure werden in 20 ml Dimethylformamid mit 0,85 g (4,4 mmol) 4-Brommethyl-5-methyl-1,3-dioxol-2-on und 0,42 g (4,2 mmol) pulverisiertes Kaliumhydrogencarbonat 3 Tage unter Eiskühlung gerührt. Die Mischung wird im Vakuum eingeengt, der Rückstand mit 20 ml Wasser verrührt, das erhaltene schmierige Produkt in Methanol gelöst und filtriert. Man läßt 2 Tage bei Raumtemperatur stehen und isoliert das ausgefallene farblose Kristallisat.

Ausbeute: 0,3 g, Schmelzpunkt: 228 - 232°C (unter Zersetzung)

Entsprechend Beispiel 1 werden folgende Verbindungen hergestellt:

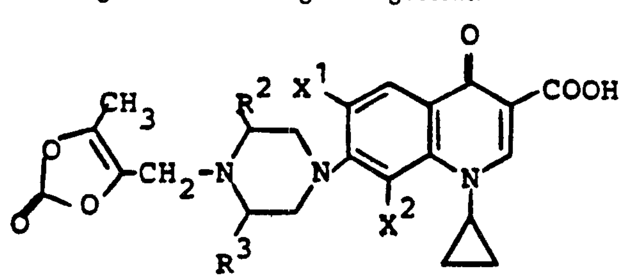

| Beispiel | $R^2$ | $R^3$ | $X^1$ | $X^2$ |
|---|---|---|---|---|
| 2 | H | H | Cl | F |
| 3 | $CH_3$ | H | Cl | F |
| 4 | $CH_3$ | $CH_3$ | Cl | F |

| | | | | |
|---|---|---|---|---|
| 5 | 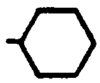 | H | Cl | F |
| 6 | 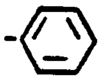 | H | Cl | F |
| 7 | H | H | F | Cl |
| 8 | $CH_3$ | H | F | Cl |
| 9 | $CH_3$ | H | Cl | Cl |
| 10 | $CH_3$ | H | F | H |
| 11 | $CH_3$ | $CH_3$ | F | H |
| 12 | 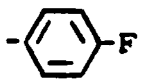 | H | F | N |
| 13 | 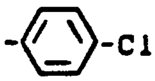 | H | F | H |
| 14 | 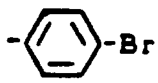 | H | F | H |
| 15 | 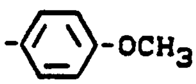 | H | F | H |
| 16 | 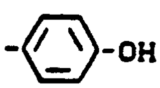 | H | F | H |
| 17 | 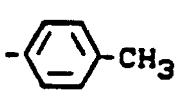 | H | F | H |
| 18 | 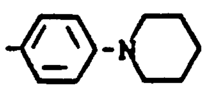 | H | F | H |
| 19 | 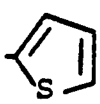 | H | F | H |
| 20 | H | H | F | F |
| 21 | $CH_3$ | H | F | F |

**Patentansprüche**

1. Cyclopropyl-1,4-dihydro-4-oxo-7-[4-(2-oxo-1,3-dioxol-4-yl-methyl)-1-piperazinyl]-3-chinolincarbonsäuren der Formel (I)

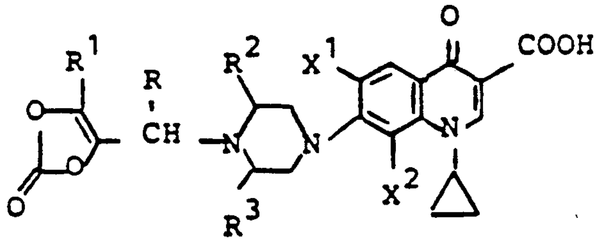

(I),

in welcher

R für ein Wasserstoffatom steht oder mit $R^1$ gemeinsam einen Alkylenrest mit 2 oder 3 Kohlenstoffatomen bildet,
$R^1$ für ein Wasserstoffatom, Alkyl mit 1 bis 4 Kohlenstoffatomen oder Phenyl steht,
$R^2$ und $R^3$ gleich oder verschieden sein können und für ein Wasserstoffatom, Methyl, Ethyl, Cyclohexyl, Methylendioxyphenyl, Furyl, Tetrahydrofuryl, Thienyl oder für gegebenenfalls durch Fluor, Chlor, Brom, Methyl, Phenyl, Cyano, Hydroxy, Methoxy, Benzyloxy, Amino, Methylamino, Dimethylamino, Piperidino oder Nitro ein- bis dreifach substituiertes Phenyl stehen,
$X^1$ für ein Wasserstoff-, Fluor-, Chlor-, Bromatom oder Nitro und
$X^2$ für ein Wasserstoff-, Fluor-, Chlor- oder Bromatom.

stehen, und deren pharmazeutisch verwendbare Hydrate, Säureadditionssalze, Alkali- und Erdalkalisalze.

2. 1-Cyclopropyl-1,4-dihydro-4-oxo-7-[4-(2-oxo-1,3-dioxol-4-yl-methyl)-1-piperazinyl]-3-chinolincarbonsäuren gemäß Anspruch 1, dadurch gekennzeichnet, daß

R für ein Wasserstoffatom steht,
$R^1$ für Methyl oder tert.-Butyl steht,
r $R^2$ und $R^3$ gleich oder verschieden sein können und für ein Wasserstoffatom, Methyl, Ethyl, Cyclohexyl, Thienyl oder für gegebenenfalls durch Fluor, Chlor, Brom, Methyl, Phenyl, Cyano, Hydroxy, Methoxy, Amino, Piperidino oder Nitro ein bis dreifach substituiertes Phenyl stehen,
$X^1$ für ein Wasserstoff-, Fluor-, Chlor-, Bromatom oder Nitro und
$X^2$ für ein Wasserstoff-, Fluor-, Chlor- oder Bromatom stehen.

3. 1-Cyclopropyl-1,4-dihydro-4-oxo-7-(4-(2-oxo-1,3-dioxol-4-yl-methyl)-1-piperazinyl]-3-chinolincarbonsäuren gemäß Anspruch 1, dadurch gekennzeichnet, daß

R für ein Wasserstoffatom steht,
$R^1$ für Methyl steht,
$R^2$ und $R^3$ gleich oder verschieden sein können und für ein Wasserstoffatom, Methyl, Cyclohexyl, Thienyl oder für gegebenenfalls durch Fluor, Chlor, Brom, Methyl, Hydroxy, Methoxy, Benzyloxy, Amino, Piperidino oder Nitro ein- oder zweifach substituiertes Phenyl stehen,
$X^1$ für ein Fluor-, Chlor- oder Bromatom und
$X^2$ für ein Wasserstoff-, Fluor-, Chlor- oder Bromatom stehen.

4. 1-Cyclopropyl-6-fluor-1,4-dihydro-7-[4-(5-methyl-2-oxo-1,3-dioxol-4-yl-methyl)-1-piperazinyl]-4-oxo-3-chinolincarbonsäure,

1-Cyclopropyl-1,4-dihydro-4-oxo-7-[4-(2-oxo-1,3-dioxol-4-yl-methyl)-1-piperazinyl]-3-chinolincarbonsäuren aus der Gruppe bestehend aus

1-Cyclopropyl-6-fluor-1,4-dihydro-4-oxo-7-[4-(2-oxo-1,3-dioxol-4-yl-methyl)-1-piperazinyl]-3-chinolincarbonsäure,

7-[4-(5-tert.-Butyl-2-oxo-1,3-dioxol-4-yl-methyl)-1-piperazinyl]-1-cyclopropyl-6-fluor-1,4-dihydro-4-oxo-3-chinolincarbonsäure,

1-Cyclopropyl-6-fluor-1,4-dihydro-4-oxo-7-[4-(2-oxo-5-phenyl-1,3-dioxol-4-yl-methyl)-1-piperazin yl]-3-chinolincarbonsäure,

1-Cyclopropyl-6-fluor-1,4-dihydro-4-oxo-7-[4-(4,5,6,7-tetrahydro-2-oxo-1,3-benzdioxol-4-yl)-1-piperazinyl]-3-chinolincarbonsäure,

1-Cyclopropyl-6-fluor-7-[3-(4-fluor-phenyl)-4-(5-methyl-2-oxo-1,3-dioxol-4-yl-methyl)-1-piperazinyl]-1,4-dihydro-4-oxo-3-chinolincarbonsäure,

1-Cyclopropyl-6-fluor-1,4-dihydro-4-oxo-7-[4-(5-methyl-2-oxo-1,3-dioxol-4-yl-methyl)-3-(2-thienyl)-1-piperazinyl]-3-chinolincarbonsäure,

1-Cyclopropyl-6,8-difluor-1,4-dihydro-7-[3-methyl-4-(5-methyl-2-oxo-1,3-dioxol-4-yl-methyl)-1-piperazinyl]-4-oxo-3-chinolincarbonsäure,

1-Cyclopropyl-6,8-difluor-1,4-dihydro-7-[3,5-dimethyl-4-(5-methyl-2-oxo-1,3-dioxol-4-yl-methyl)-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-phenyl-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-(2-thienyl)-1-piperazinyl]-4-oxo-3-chinolincarbonsäure,
6-Chlor-7-[3-cyclohexyl-4-(5-methyl-2-oxo-1,3-dioxol-4-yl-methyl)-1-piperazinyl]-1-cyclopropyl-8-fluor-1,4-dihydro-4-oxo-3-chinolincarbonsäure,
8-Chlor-1-cyclopropyl-6-fluor-1,4-dihydro-7-[4-(5-ethyl-2-oxo-1,3-dioxol-4-yl-methyl)-1-piperazinyl]-4-oxo-3-chinolincarbonsäure,
8-Chlor-1-cyclopropyl-6-fluor-1,4-dihydro-7-[3-methyl-4-(5-methyl-2-oxo-1,3-dioxol-4-yl-methyl)-1-piperazinyl]-4-oxo-3-chinolincarbonsäure, und
6,8-Dichlor-1-cyclopropyl-1,4-dihydro-7-[3-methyl-4-(5-methyl-2-oxo-1,3-dioxol-4-yl-methyl)-1-piperazinyl]-4-oxo-3-chinolincarbonsäure,
6-Chlor-1-cyclopropyl-8-fluor-1,4-dihydro-7-[4-(5-methyl-2-oxo-1,3-dioxol-4-yl-methyl)-1-piperazinyl]-4-oxo-3-chinolincarbonsäure,
6-Chlor-1-cyclopropyl-8-fluor-1,4-dihydro-7-[3-methyl-4-(5-methyl-2-oxo-1,3-dioxol-4-yl-methyl)-1-piperazinyl]-4-oxo-3-chinolincarbonsäure,
6-Chlor-1-cyclopropyl-8-fluor-1,4-dihydro-7-[3,5-dimethyl-4-(5-methyl-2-oxo-1,3-dioxol-4-yl-methyl)-1-piperazinyl]-4-oxo-3-chinolincarbonsäure,
6-Chlor-1-cyclopropyl-8-fluor-1,4-dihydro-7-[4-(5-methyl-2-oxo-1,3-dioxol-4-yl-methyl)-3-phenyl-1-piperazinyl]-4-oxo-3-chinolincarbonsäure,
1-Cyclopropyl-6-fluor-1,4-dihydro-7-[3-methyl-4-(5-methyl-2-oxo-1,3-dioxol-4-yl-methyl)-1-piperazinyl]-4-oxo-3-chinolincarbonsäure,
1-Cyclopropyl-6-fluor-1,4-dihydro-7-[3,5-dimethyl-4-(5-methyl-2-oxo-1,3-dioxol-4-yl-methyl)-1-piperazinyl]-4-oxo-3-chinolincarbonsäure,
7-[3-(4-Chlorphenyl)-4-(5-methyl-2-oxo-1,3-dioxol-4-yl-methyl)-1-piperazinyl]-1-cyclopropyl-6-fluor-1,4-dihydro-4-oxo-3-chinolincarbonsäure,
7-[3-(4-Bromphenyl)-4-(5-methyl-2-oxo-1,3-dioxol-4-yl-methyl)-1-piperazinyl]-1-cyclopropyl-6-fluor-1,4-dihydro-4-oxo-3-chinolincarbonsäure,
1-Cyclopropyl-6-fluor-1,4-dihydro-7-[3-(4-methoxyphenyl)-4-(5-methyl-2-oxo-1,3-dioxol-4-yl-methyl)-1-piperazinyl]-4-oxo-3-chinolincarbonsäure,
1-Cyclopropyl-6-fluor-1,4-dihydro-7-[3-(4-hydroxyphenyl)-4-(5-methyl-2-oxo-1,3-dioxol-4-yl-methyl)-1-piperazinyl]-4-oxo-3-chinolincarbonsäure,
1-Cyclopropyl-6-fluor-1,4-dihydro-7-[4-(5-methyl-2-oxo-1,3-dioxol-4-yl-methyl)-3-(4-methylphenyl)-1-piperazinyl]-4-oxo-3-chinolincarbonsäure,
1-Cyclopropyl-6-fluor-1,4-dihydro-7-[4-(5-methyl-2-oxo-1,3-dioxol-4-yl-methyl)-3-(4-piperidino-phenyl)-1-piperazinyl]-4-oxo-3-chinolincarbonsäure,
1-Cyclopropyl-6,8-difluor-1,4-dihydro-7-[4-(5-methyl-2-oxo-1,3-dioxol-4-yl-methyl)-1-piperazinyl]-4-oxo-3-chinolincarbonsäure.

5. Verfahren zur Herstellung von 1-Cyclopropyl-1,4-dihydro-4-oxo-7-[4-(2-oxo-1,3-dioxol-4-yl)-methyl-1-piperazinyl]-3-chinolincarbonsäuren der Formel (I) nach Anspruch 1

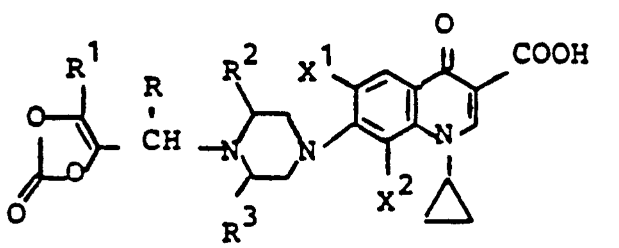

(I),

in welcher
R, $R^1$, $R^2$, $R^3$, $X^1$ und $X^2$ die in Anspruch 1 angegebene Bedeutung haben, dadurch gekennzeichnet, daß man 1-Cyclopropyl-1,4-dihydro-4-oxo-7-(1-piperazinyl)-3-chinolincarbonsäuren der Formel (II)

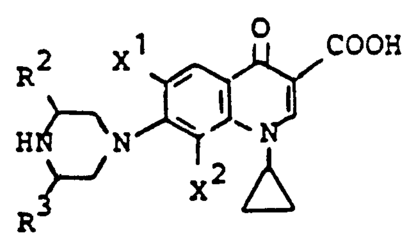

(II),

in welcher $R^2$, $R^3$, $X^1$ und $X^2$ die in Anspruch 1 angegebene Bedeutung haben, mit Verbindungen der Formel (III)

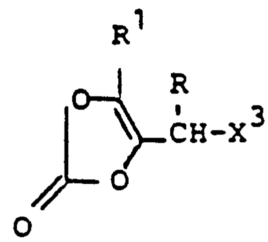

(III),

in welcher $R^1$ und R die in Anspruch 1 angegebene Bedeutung haben und

$X^3$ für ein Chlor-, Brom- oder Jodatom oder einen Rest $O\text{-}SO_2\text{-}R^4$ steht, wobei

$R^4$ Methyl, Trifluormethyl, Phenyl oder 4-Methylphenyl bedeutet,

gegebenenfalls in Gegenwart von Säurebindern umsetzt.

6. 1-Cyclopropyl-1,4-dihydro-4-oxo-7-[4-(2-oxo-1,3-dioxol-4-yl-methyl)-1-piperazinyl]-3-chinolincarbonsäuren der Formel (I) nach Anspruch 1

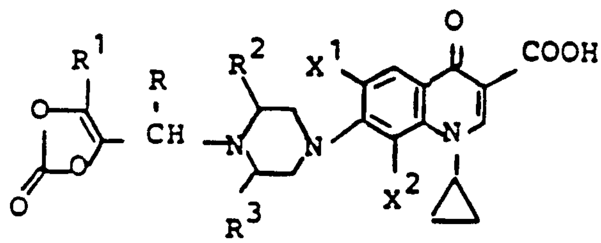

(I),

in welcher

R, $R^1$, $R^2$, $R^3$, $X^1$ und $X^2$ die in Anspruch 1 angegebene Bedeutung haben, zur Anwendung bei der therapeutischen Behandlung des menschlichen oder tierischen Körpers.

7. Arzneimittel enthaltend 1-Cyclopropyl-1,4-dihydro-4-oxo-7[4-(2-oxo-1,3-dioxol-4-yl-methyl)-1-piperazinyl]-3-chinolincarbonsäuren der Formel (I) nach Anspruch 1

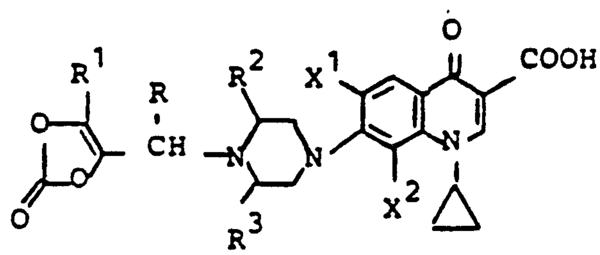

(I)

in welcher

R, $R^1$, $R^2$, $R^3$, $X^1$ und $X^2$ die in Anspruch 1 angegebene Bedeutung haben, und deren pharmazeutisch verwendbaren Hydrate, Säureadditionssalze, Alkali- und Erdalkalisalze.

8. Verwendung von 1-Cyclopropyl-1,4-dihydro-4-oxo-7-[4-(2-oxo-1,3-dioxol 4-yl-methyl)-1-piperazinyl]-3-chinolincarbonsäuren der Formel (I) nach Anspruch 1

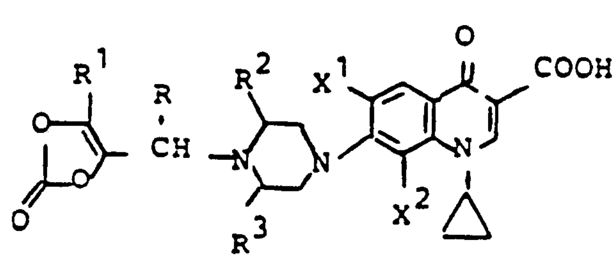

(I)

in welcher

R, $R^1$, $R^2$, $R^3$, $X^1$ und $X^2$ die in Anspruch 1 angegebene Bedeutung haben, und deren pharmazeutisch verwendbaren Hydraten, Säureadditionssalzen, Alkali- und Erdalkalisalzen zur

Herstellung von Arzneimitteln.

9. Verwendung von 1-Cyclopropyl-6-fluor-1,4-dihydro-7-[4-(5-methyl-2-oxo-1,3-dioxol-4-yl-methyl)-1-piperazinyl]-4-oxo-3-chinolincarbonsäure,

1-Cyclopropyl-6-fluor-1,4-dihydro-4-oxo-7-[4-(2-oxo-1,3-dioxol-4-yl-methyl)-1-piperazinyl]-3-chinolincarbonsäure,

7-[4-(5-tert.-Butyl-2-oxo-1,3-dioxol-4-yl-methyl)-1-piperazinyl]-1-cyclopropyl-6-fluor-1,4-dihydro-4-oxo-3-chinolincarbonsäure,

1-Cyclopropyl-6-fluor-1,4-dihydro-4-oxo-7-[4-(2-oxo-5-phenyl-1,3-dioxol-4-yl-methyl)-1-piperazinyl]-3-chinolincarbonsäure,

1-Cyclopropyl-6-fluor-1,4-dihydro-4-oxo-7-[4-(4,5,6,7-tetrahydro-2-oxo-1,3-benzdioxol-4-yl)-1-piperazinyl]-3-chinolincarbonsäure,

1-Cyclopropyl-6-fluor-7-[3-(4-fluor-phenyl)-4-(5-methyl-2-oxo-1,3-dioxol-4-yl-methyl)-1-piperazinyl]-1,4-dihydro-4-oxo-3-chinolincarbonsäure,

1-Cyclopropyl-6-fluor-1,4-dihydro-4-oxo-7-[4-(5-methyl-2-oxo-1,3-dioxol-4-yl-methyl)-3-(2-thienyl)-1-piperazinyl]-3-chinolincarbonsäure,

1-Cyclopropyl-6,8-difluor-1,4-dihydro-7-[3-methyl-4-(5-methyl-2-oxo-1,3-dioxol-4-yl-methyl)-1-piperazinyl]-4-oxo-3-chinolincarbonsäure,

1-Cyclopropyl-6,8-difluor-1,4-dihydro-7-[3,5-dimethyl-4-(5-methyl-2-oxo-1,3-dioxol-4-yl-methyl)-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-phenyl-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-3-(2-thienyl)-1-piperazinyl)-4-oxo-3-chinolincarbonsäure,

6-Chlor-7-[3-cyclohexyl-4-(5-methyl-2-oxo-1,3-dioxol-4-yl-methyl)-1-piperazinyl]-1-cyclopropyl-8-fluor-1,4-dihydro-4-oxo-3-chinolincarbonsäure,

8-Chlor-1-cyclopropyl-6-fluor-1,4-dihydro-7-[4-(5-methyl-2-oxo-1,3-dioxol-4-yl-methyl)-1-piperazinyl]-4-oxo-3-chinolincarbonsäure,

8-Chlor-1-cyclopropyl-6-fluor-1,4-dihydro-7-[3-methyl-4-(5-methyl-2-oxo-1,3-dioxol-4-yl-methyl)-1-piperazinyl]-4-oxo-3-chinolincarbonsäure,

6,8-Dichlor-1-cyclopropyl-1,4-dihydro-7-[3-methyl-4-(5-methyl-2-oxo-1,3-dioxol-4-yl-methyl)-1-piperazinyl]-4-oxo-3-chinolincarbonsäure,

6-Chlor-1-cyclopropyl-8-fluor-1,4-dihydro-7-[4-(5-methyl-2-oxo-1,3-dioxol-4-yl-methyl)-1-piperazinyl]-4-oxo-3-chinolincarbonsäure,

6-Chlor-1-cyclopropyl-8-fluor-1,4-dihydro-7-[3-methyl-4-(5-methyl-2-oxo-1,3-dioxol-4-yl-methyl)-1-piperazinyl]-4-oxo-3-chinolincarbonsäure,

6-Chlor-1-cyclopropyl-8-fluor-1,4-dihydro-7-[3,5-dimethyl-4-(5-methyl-2-oxo-1,3-dioxol-4-yl-methyl)-1-piperazinyl]-4-oxo-3-chinolincarbonsäure,

6-Chlor-1-cyclopropyl-8-fluor-1,4-dihydro-7-[4-(5-methyl-2-oxo-1,3-dioxol-4-yl-methyl)-3-phenyl-1-piperazinyl]-4-oxo-3-chinolincarbonsäure,

1-Cyclopropyl-6-fluor-1,4-dihydro-7-[3-methyl-4-(5-methyl-2-oxo-1,3-dioxol-4-yl-methyl)-1-piperazinyl]-4-oxo-3-chinolincarbonsäure,

1-Cyclopropyl-6-fluor-1,4-dihydro-7-(3,5-dimethyl-4-(5-methyl-2-oxo-1,3-dioxol-4-yl-methyl)-1-piperazinyl]-4-oxo-3-chinolincarbonsäure,

7-[3-(4-Chlorphenyl)-4-(5-methyl-2-oxo-1,3-dioxol-4-yl-methyl)-1-piperazinyl]-1-cyclopropyl-6-fluor-1,4-dihydro-4-oxo-3-chinolincarbonsäure,

7-[3-(4-Bromphenyl)-4-(5-methyl-2-oxo-1,3-dioxol-4-yl-methyl)-1-piperazinyl]-1-cyclopropyl-6-fluor-1,4-dihydro-4-oxo-3-chinolincarbonsäure,

1-Cyclopropyl-6-fluor-1,4-dihydro-7-[3-(4-methoxyphenyl)-4-(5-methyl-2-oxo-1,3-dioxol-4-yl-methyl)-1-piperazinyl]-4-oxo-3-chinolincarbonsäure,

1-Cyclopropyl-6-fluor-1,4-dihydro-7-[3-(4-hydroxyphenyl)-4-(5-methyl-2-oxo-1,3-dioxol-4-yl-methyl)-1-piperazinyl]-4-oxo-3-chinolincarbonsäure,

1-Cyclopropyl-6-fluor-1,4-dihydro-7-[4-(5-methyl-2-oxo-1,3-dioxol-4-yl-methyl)-3-(4-methylphenyl)-1-piperazinyl]-4-oxo-3-chinolincarbonsäure,

1-Cyclopropyl-6-fluor-1,4-dihydro-7-[4-(5-methyl-2-oxo-1,3-dioxol-4-yl-methyl)-3-(4-piperidino-phenyl)-1-piperazinyl]-4-oxo-3-chinolincarbonsäure,

1-Cyclopropyl-6,8-difluor-1,4-dihydro-7-[4-(5-methyl-2-oxo-1,3-dioxol-4-yl-methyl)-1-piperazinyl]-4-oxo-3-chinolincarbonsäure zur Herstellung von Arzneimitteln.

10. 1-Cyclopropyl-1,4-dihydro-7-[4-(2-oxo-1,3-dioxol-4-yl-methyl)-1-piperazinyl]-3-chinolin-carbonsäuren aus der Gruppe bestehend aus 1-Cyclopropyl-6-fluor-1,4-dihydro-4-oxo-7-[4-(5-methyl-2-oxo-1,3-dioxol-4-yl-methyl)-1-piperazinyl]-4-oxo-3-chinolincarbonsäure;

8-Chlor-1-cyclopropyl-6-fluor-1,4-dihydro-7-[4-(5-methyl-2-oxo-1,3-dioxol-4-yl-methyl)-1-piperazinyl]-4-oxo-3-chinolincarbonsäure;

8-Chlor-1-cyclopropyl-6-fluor-1,4-dihydro-7-[3-methyl-4-(5-methyl-2-oxo-1,3-dioxol-4-yl-methyl)-1-piperazinyl]-4-oxo-3-chinolincarbonsäure;

1-Cyclopropyl-6,8-difluor-1,4-dihydro-7-[4-(5-methyl-2-oxo-1,3-dioxol-4-yl-methyl)-1-piperazinyl]-4-oxo-3-

chinolincarbonsäure, und

1-Cyclopropyl-6,8-difluor-1,4-dihydro-7-[3-methyl-4-(5-methyl-2-oxo-1,3-dioxol-4-yl-methyl)-1-piperazinyl]-4-oxo-3-chinolincarbonsäure.

**Claims**

1. 1-Cyclopropyl-1,4-dihydro-4-oxo-7-[4-(2-oxo-1,3-dioxol-4-yl-methyl)-1-piperazinyl]-3-quinolinecarboxylic acids of the formula (I)

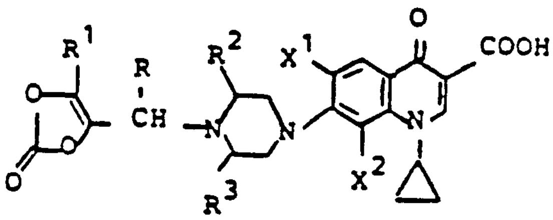

(I),

in which

| | |
|---|---|
| R | represents a hydrogen atom or, together with $R^1$, forms an alkylene radical with 2 or 3 carbon atoms, |
| $R^1$ | represents a hydrogen atom, alkyl with 1 to 4 carbon atoms or phenyl, |
| $R^2$ and $R^3$ | can be identical or different and represent a hydrogen atom, methyl, ethyl, cyclohexyl, methylenedioxyphenyl, furyl, tetrahydrofuryl or thienyl, or represent phenyl which is optionally mono-, di- or trisubstituted by fluorine, chlorine, bromine, methyl, phenyl, cyano, hydroxyl, methoxy, benzyloxy, amino, methylamino, dimethylamino, piperidino or nitro, |
| $X^1$ | represents a hydrogen, fluorine, chlorine or bromine atom, or nitro and |
| $X^2$ | represents a hydrogen, fluorine, chlorine or bromine atom, |

and pharmaceutically usable hydrates, acid addition salts and alkali metal and alkaline earth metal salts thereof.

2. 1-Cyclopropyl-1,4-dihydro-4-oxo-7-[4-(2-oxo-1,3-dioxol-4-yl-methyl)-1-piperazinyl]-3-quinolinecarboxylic acids according to Claim 1, characterized in that

| | |
|---|---|
| R | represents a hydrogen atom, |
| $R^1$ | represents methyl or tert.-butyl, |
| $R^2$ and $R^3$ | can be identical or different and represent a hydrogen atom, methyl, ethyl, cyclohexyl or thienyl or represent phenyl which is optionally mono-, di- or tri-substituted by fluorine, chlorine, bromine, methyl, phenyl, cyano, hydroxyl, methoxy, amino, piperidino or nitro, |
| $X^1$ | represents a hydrogen, fluorine, chlorine or bromine atom, or nitro and |
| $X^2$ | represents a hydrogen, fluorine, chlorine or bromine atom. |

3. 1-Cyclopropyl-1,4-dihydro-4-oxo-7-[4-(2-oxo-1,3-dioxol-4-yl-methyl)-1-piperazinyl]-3-quinolinecarboxylic acids according to Claim 1, characterized in that

| | |
|---|---|
| R | represents a hydrogen atom, |
| $R^1$ | represents methyl, |
| $R^2$ and $R^3$ | can be identical atom, methyl, cyclohexyl represent a hydrogen atom, methyl, cyclohexyl or thienyl, or represent phenyl which is optionally mono- or di-substituted by fluorine, chlorine, bromine, methyl, hydroxyl, methoxy, benzyloxy, amino, piperidino or nitro, |
| $X^1$ | represents a fluorine, chlorine or bromine atom, and |
| $X^2$ | represent a hydrogen, fluorine, chlorine or bromine atom. |

4. 1-Cyclopropyl-6-fluoro-1,4-dihydro-7-[4-(5-methyl-2-oxo-1,3-dioxol-4-yl-methyl)-1-piperazinyl]-4-oxo-3-quinolinecarboxylic acid, 1-cyclopropyl-1,4-dihydro-4-oxo-7-[4-(2-oxo-1,3-dioxol-4-yl-methyl)-1-piperazinyl]-3-quinolinecarboxylic acids from the group consisting of 1-cyclopropyl-6-fluoro-1,4-dihydro-4-oxo-7-[4-(2-oxo-1,3-dioxol-4-yl-methyl)-1-piperazinyl]-3-quinolinecarboxylic acid, 7-[4-(5-tert.-butyl-2-oxo-1,3-dioxol-4-yl-methyl)-1-piperazinyl]-1-cyclopropyl-6-fluoro-1,4-dihydro-4-oxo-3-quinolinecarboxylic acid, 1-cyclopropyl-6-fluoro-1,4-dihydro-4-oxo-7-[4-(2-oxo-5-phenyl-1,3-dioxol-4-yl-methyl)-1-piperazinyl]-3-quinolinecarboxylic acid, 1-cyclopropyl-6-fluoro-1,4-dihydro-4-oxo-7-[4-(4,5,6,7-tetrahydro-2-oxo-1,3-benzdioxol-4-yl)-1-piperazinyl]-3-quinolinecarboxylic acid, 1-cyclopropyl-6-fluoro-7-[3-(4-fluoro-phenyl)-4-(5-methyl-2-oxo-1,3-dioxol-4-yl-methyl)-1-piperazinyl]-1,4-dihydro-4-oxo-3-quinolinecarboxylic acid, 1-cyclopropyl-6-fluoro-1,4-dihydro-4-oxo-7-[4-(5-methyl-2-oxo-1,3-dioxol-4-yl-methyl)-3-(2-thienyl)-1-piperazinyl]-3-quinolinecarboxylic acid, 1-cyclopropyl-6,8-difluoro-1,4-dihydro-7-[3-methyl-4-(5-methyl-2-oxo-1,3-dioxol-4-yl-methyl)-1-piperazinyl]-4-oxo-3-quinolinecarboxylic acid, 1-cyclopropyl-6,8-difluoro-1,4-di-hydro-7-[3,5-dimethyl-4-(5-methyl-2-oxo-1,3-dioxol-4-yl-methyl)-1-piperazinyl]-4-oxo-3-quinolinecarboxylic acid, 1-cyclopropyl-6,8-difluoro-1,4-dihydro-7-[4-(5-methyl-2-oxo-1,3-dioxol-4-yl-methyl)-3-phenyl-1-piperazinyl]-4-oxo-3-quinolinecarboxylic acid, 1-cyclopropyl-6,8-difluoro-1,4-dihydro-7-[4-(5-methyl-2-oxo-1,3-dioxol-4-yl-methyl)-3-(2-thienyl)-1-piperazinyl]-4-oxo-3-quinolinecarboxylic acid, 6-chloro-7-[3-cyclohexyl-4-(5-methyl-2-oxo-1,3-dioxol-4-yl-methyl)-1-piperazinyl]-1-cyclopropyl-6-fluoro-1,4-dihydro-4-oxo-3-quinolinecarboxylic acid, 8-chloro-1-cyclopropyl-8-fluoro-1,4-dihydro-7-[4-(5-ethyl-2-oxo-1,3-dioxol-4-yl-methyl)-1-piperazinyl]-4-oxo-3-quinolinecarboxylic acid, 8-chloro-1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid and 6,8-dichloro-1-cyclopropyl-1,4-dihydro-7-[3-methyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[3-methyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[3,5-dimethyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[4-(5-methyl-2-oxo-1,3-dioxol-4-yl-methyl)-3-phenyl-1-piperazinyl]-4-oxo-3-quinolinecarboxylic acid, 1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-7-[3,5-dimethyl-4-(5-methyl-2-oxo-1,3-dioxol-4-yl-methyl)-1-piperazinyl]-4-oxo-3-quinolinecarboxylic acid, 7-[3-(4-chlorophenyl)-4-(5-methyl-2-oxo-1,3-dioxol-4-yl-methyl)-1-piperazinyl]-1-cyclopropyl-6-fluoro-1,4-dihydro-4-oxo-3-quinolinecarboxylic acid, 7-[3-(4-bromophenyl)-4-(5-methyl-2-oxo-1,3-dioxol-4-yl-methyl)-1-piperazinyl]-1-cyclopropyl-6-fluoro-1,4-dihydro-4-oxo-3-quinolinecarboxylic acid, 1-cyclopropyl-6-fluoro-1,4-dihydro-7-[3-(4-methoxyphenyl)-4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-7-[3-(4-hydroxyphenyl)-4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-7-[4-(5-methyl-2-oxo-1,3-dioxol-4-yl-methyl)-3-(4-methylphenyl)-1-piperazinyl]-4-oxo-3-quinolinecarboxylic acid, 1-cyclopropyl-6-fluoro-1,4-dihydro-7-[4-(5-methyl-2-oxo-1,3-dioxol-4-yl-methyl)-3-(4-piperidino-phenyl)-1-piperazinyl]-4-oxo-3-quinolinecarboxylic acid and 1-cyclopropyl-6,8-difluoro-1,4-dihydro-7-[4-(5-methyl-2-oxo-1,3-dioxol-4-yl-methyl)-1-piperazinyl]-4-oxo-3-quinolinecarboxylic acid.

5. Process for the preparation of 1-cyclopropyl-1,4-dihydro-4-oxo-7-[4-(2-oxo-1,3-dioxol-4-yl)-methyl-1-piperazinyl]-3-quinolinecarboxylic acids of the formula (I) according to Claim 1

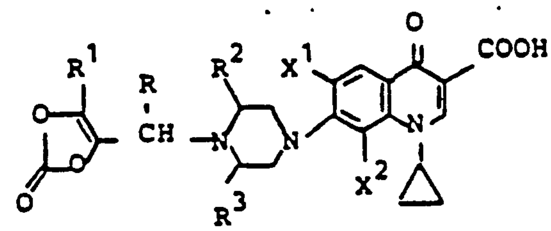

(I),

in which

R, $R^1$, $R^2$, $R^3$, $X^1$ and $X^2$ have the meaning given in Claim 1,

characterized in that 1-cyclopropyl-1,4-dihydro-4-oxo-7-(1-piperazinyl)-3-quinolinecarboxylic acids of the formula (II)

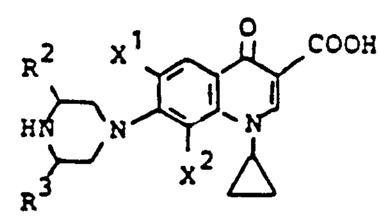

(II),

in which

$R^2$, $R^3$, $X^1$ and $X^2$ have the meaning given in Claim 1, are reacted with compounds of the formula (III)

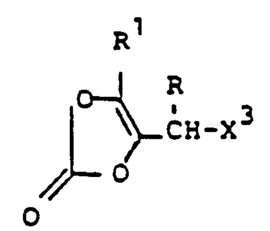

(III),

in which

$R^1$ and R have the meaning given in Claim 1 and

$X^3$ represents a chlorine, bromine or iodine atom, or a radical $O-SO_2-R^4$,
wherein
$R^4$ denotes methyl, trifluoromethyl, phenyl or 4-phenylmethylphenyl,
if appropriate in the presence of acid-binding agents.

6. 1-Cyclopropyl-1,4-dihydro-4-oxo-7-[4-(2-oxo-1,3-dioxol-4-yl-methyl)-1-piperazinyl]-3-quinolinecarboxylic acids of the formula (I) according to Claim 1

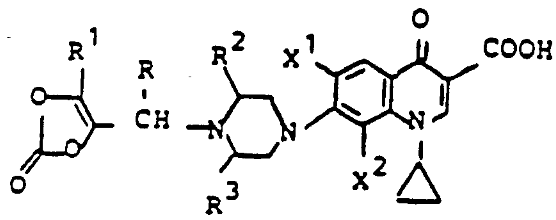

(I),

in which
R, $R^1$, $R^2$, $R^3$, $X^1$ and $X^2$ have the meaning given in Claim 1,
for use in the therapeutic treatment of the human or animal body.

7. Medicaments containing 1-cyclopropyl-1,4-dihydro-4-oxo-7-[4-(2-oxo-1,3-dioxol-4-yl-methyl)-1-piperazinyl]-3-quinolinecarboxylic acids of the formula (I) according to Claim 1

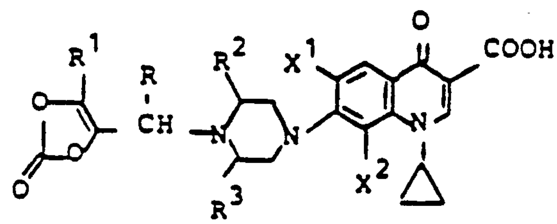

(I)

in which
R, $R^1$, $R^2$, $R^3$, $X^1$ and $X^2$ have the meaning given in Claim 1,
and pharmaceutically usable hydrates, acid addition salts and alkali metal and alkaline earth metal salts thereof.

8. Use of 1-cyclopropyl-1,4-dihydro-4-oxo-7-[4-(2-oxo-1,3-dioxol-4-yl-methyl)-1-piperazinyl]-3-quinolinecarboxylic acids of the formula (I) according to Claim

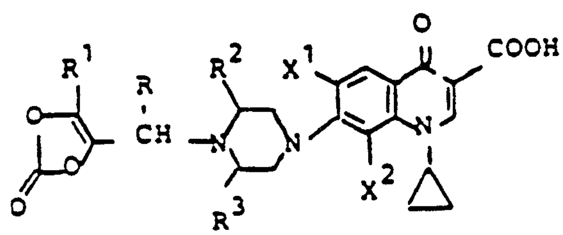

(I)

in which
R, $R^1$, $R^2$, $R^3$, $X^1$ and $X^2$ have the meaning given in Claim 1,
and pharmaceutically usable hydrates, acid addition salts and alkali metal and alkaline earth metal salts thereof, for the preparation of medicaments.

9. Use of 1-cyclopropyl-6-fluoro-1,4-dihydro-7-[4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-4-oxo-7-[4-(2-oxo-1,3-dioxol-4-yl-methyl)-1-piperazinyl]-3-quinolinecarboxylic acid, 7-[4-(5-tert.-butyl-2-oxo-1,3-dioxol-4-yl-methyl)-1-piperazinyl]-1-cyclopropyl-6-fluoro-1,4-dihydro-4-oxo-3-quinolinecarboxylic acid, 1-cyclopropyl-6-fluoro-1,4-dihydro-4-oxo-7-[4-(2-oxo-5-phenyl-1,3-dioxol-4-yl-methyl)-1-piperazinyl]-3-quinolinecarboxylic acid, 1-cyclopropyl-6-fluoro-1,4-dihydro-4-oxo-7-[4-(4,5,6,7-tetrahydro-2-oxo-1,3-benzdioxol-4-yl)-1-piperazinyl]-3-quinolinecarboxylic acid, 1-cyclopropyl-6-fluoro-7-[3-(4-fluoro-phenyl)-4-(5-methyl-2-oxo-1,3-dioxol-4-yl-methyl)-1-piperazinyl]-1,4-dihydro-4-oxo-3-quinolinecarboxylic acid, 1-cyclopropyl-6-fluoro-1,4-dihydro-4-oxo-7-[4-(5-methyl-2-oxo-1,3-dioxol-4-yl-methyl)-3-(2-thienyl)-1-piperazinyl]-3-quinolinecarboxylic acid, 1-cyclopropyl-6,8-difluoro-1,4-dihydro-7-[3-methyl-4-(5-methyl-2-oxo-1,3-dioxol-4-yl-methyl)-1-piperazinyl]-4-oxo-3-quinolinecarboxylic acid, 1-cyclopropyl-6,8-difluoro-1,4-dihydro-7-[3,5-dimethyl-4-(5-methyl-2-oxo-1,3-dioxol-4-yl-methyl)-1-piperazinyl]-4-oxo-3-quinolinecarboxylic acid, 1-cyclopropyl-6,8-difluoro-1,4-dihydro-7-[4-(5-methyl-2-oxo-1,3-dioxol-4-yl-methyl)-3-phenyl-1-piperazinyl]-4-oxo-3-quinolinecarboxylic acid, 1-cyclopropyl-6,8-difluoro-1,4-dihydro-7-[4-(5-methyl-2-oxo-1,3-dioxol-4-yl-methyl)-3-(2-thienyl)-1-piperazinyl]-4-oxo-3-quinolinecarboxylic acid, 6-chloro-7-[3-cyclohexyl-4-(5-methyl-2-oxo-1,3-dioxol-4-yl-methyl)-1-piperazinyl]-1-cyclopropyl-8-fluoro-1,4-dihydro-4-oxo-3-quinolinecarboxylic acid, 8-chloro-1-cyclopropyl-6-fluoro-1,4-dihydro-7-[4-(5-methyl-2-oxo-1,3-dioxol-4-yl-methyl)-1-piperazinyl]-4-oxo-3-quinolinecarboxylic acid, 8-chloro-1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid, 6,8-dichloro-1-cyclopropyl-1,4-dihydro-7-[3-methyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[4-(5-methyl-2-oxo-1,3-dioxol-4-yl-methyl)-1-piperazinyl]-4-oxo-3-quinolinecarboxylic acid, 6-chloro-1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[3,5-dimethyl-4-(5-methyl-2-oxo-1,3-dioxol-4-yl-methyl)-1-piperazinyl]-4-oxo-3-quinolinecarboxylic acid, 6-chloro-1-cyclopropyl-8-fluoro-1,4-dihydro-7-[4-(5-methyl-2-oxo-1,3-dioxol-4-yl-methyl)-3-phenyl-1-piperazinyl]-4-oxo-3-quinolinecarboxylic acid, 1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-7-[3,5-dimethyl-4-(5-methyl-2-oxo-1,3-dioxol-4-yl-methyl)-1-piperazinyl]-4-oxo-3-quinolinecarboxylic acid, 7-[3-(4-chlorophenyl)-4-(5-methyl-2-oxo-1,3-dioxol-4-yl-methyl)-1-piperazinyl]-1-cyclopropyl-6-fluoro-1,4-dihydro-4-oxo-3-quinolinecarboxylic acid, 7-[3-(4-bromophenyl)-4-(5-methyl-2-oxo-1,3-dioxol-4-yl-methyl)-1-piperazinyl]-1-cyclopropyl-6-fluoro-1,4-dihydro-4-oxo-3-quinolinecarboxylic acid, 1-cyclopropyl-6-fluoro-1,4-dihydro-7-[3-(4-methoxyphenyl)-4-(5-methyl-2-oxo-1,3-dioxol-4-yl-methyl)-1-piperazinyl]-4-oxo-3-quinolinecarboxylic acid, 1-cyclopropyl-6-fluoro-1,4-dihydro-7-[3-(4-hydroxyphenyl)-4-(5-methyl-2-oxo-1,3-dioxol-4-yl-methyl)-1-piperazinyl]-4-oxo-3-quinolinecarboxylic acid, 1-cyclopropyl-8-fluoro-1,4-dihydro-7-[4-(5-methyl-2-oxo-1,3-dioxol-4-yl-methyl)-3-(4-methylphenyl)-1-piperazinyl]-4-oxo-3-quinolinecarboxylic acid, 1-cyclopropyl-6-fluoro-1,4-dihydro-7-[4-(5-methyl-2-oxo-1,3-dioxol-4-yl-methyl)-3-(4-piperidinophenyl)-1-piperazinyl]-4-oxo-3-quinolinecarboxylic acid and 1-cyclopropyl-6,8-difluoro-1,4-dihydro-7-[4-(5-methyl-2-oxo-1,3-dioxol-4-yl-methyl)-1-piperazinyl]-4-oxo-3-quinolinecarboxylic acid for the preparation of medicaments.

10. 1-Cyclopropyl-1,4-dihydro-4-oxo-7-[4-(2-oxo-1,3-dioxol-4-yl-methyl)-1-piperazinyl]-3-quinolinecarboxylic acids from the group consisting of 1-cyclopropyl-6-fluoro-1,4-dihydro-7-[4-(5-methyl-2-oxo-1,3-dioxol-4-yl-methyl)-1-piperazinyl]-4-oxo-3-quinolinecarboxylic acid; 8-chloro-1-cyclopropyl-6-fluoro-1,4-dihydro-7-[4-(5-methyl-2-oxo-1,3-dioxol-4-yl-methyl)-1-piperazinyl]-4-oxo-3-quinolinecarboxylic acid; 8-chloro-1-cyclopropyl-6-fluoro-1,4-dihydro-7-[3-methyl-4-(5-methyl-2-oxo-1,3-dioxol-4-yl-methyl)-1-piperazinyl]-4-oxo-3-quinolinecarboxylic acid; 1-cyclopropyl-6,8-difluoro-1,4-dihydro-7-[4-(5-methyl-2-oxo-1,3-dioxol-4-yl-methyl)-1-piperazinyl]-4-oxo-3-quinolinecarboxylic acid, and 1-cyclopropyl-6,8-difluoro-1,4-dihydro-7-[3-methyl-4-(5-methyl-2-oxo-1,3-dioxol-4-yl-methyl)-1-piperazinyl]-4-oxo-3-quinolinecarboxylic acid.

## Revendications

1. Acides 1-cyclopropyl-1,4-dihydro-4-oxo-7-[4-(2-oxo-1,3-dioxol-4-yl-méthyl)-1-pipérazinyl]-3-quinoléine-carboxyliques de formule (I):

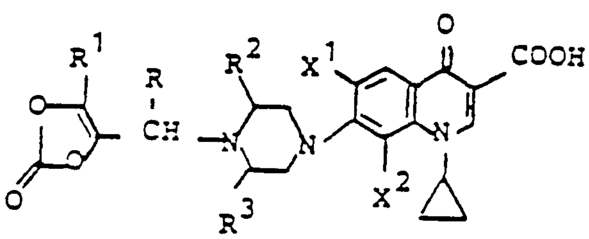

(I),

dans laquelle

| | |
|---|---|
| R | représente un atome d'hydrogène ou, ensemble avec $R^1$, il forme un groupe alkylène contenant 2 ou 3 atomes de carbone, |
| $R^1$ | représente un atome d'hydrogène, un groupe alkyle contenant 1 à 4 atomes de carbone ou un groupe phényle, |
| $R^2$ et $R^3$ | peuvent être identiques ou différents et représentent chacun un atome d'hydrogène, un groupe méthyle, un groupe éthyle, un groupe cyclohexyle, un groupe méthylène-dioxyphényle, un groupe furyle, un groupe tétrahydrofuryle, un groupe thiényle ou un groupe phényle éventuellement substitué une à trois fois par un atome de fluor, un atome de chlore, un atome de brome, un groupe méthyle, un groupe phényle, un groupe cyano, un groupe hydroxyle, un groupe méthoxy, un groupe benzyloxy, un groupe amino, un groupe méthylamino, un groupe diméthylamino, un groupe pipéridino ou un groupe nitro, |
| $X^1$ | représente un atome d'hydrogène, un atome de fluor, un atome de chlore, un atome de brome ou un groupe nitro, et |
| $X^2$ | représente un atome d'hydrogène, un atome de fluor, un atome de chlore ou un atome de brome, |

ainsi que leurs sels de métaux alcalins et alcalino-terreux, leurs sels d'addition d'acide et leurs hydrates pharmaceutiquement utiles.

2. Acides 1-cyclopropyl-1,4-dihydro-4-oxo-7-[-4-(2-oxo-1,3-dioxol-4-yl-méthyl)-1-pipérazinyl]-3-quinoléine-carboxyliques selon la revendication 1, caractérisés en ce que

R représente un atome d'hydrogène,
$R^1$ représente un groupe méthyle ou un groupe tert.-butyle,
$R^2$ et $R^3$ peuvent être identiques ou différents et représentent chacun un atome d'hydrogène, un groupe méthyle, un groupe éthyle, un groupe cyclohexyle, un groupe thiényle ou un groupe phényle éventuellement substitué une à trois fois par un atome de fluor, un atome de chlore, un atome de brome, un groupe méthyle, un groupe phényle, un groupe cyano, un groupe hydroxyle, un groupe méthoxy, un groupe amino, un groupe pipéridino ou un groupe nitro,
$X^1$ représente un atome d'hydrogène, un atome de fluor, un atome de chlore, un atome de brome ou un groupe nitro, et
$X^2$ représente un atome d'hydrogène, un atome de fluor, un atome de chlore ou un atome de brome.

3. Acides 1-cyclopropyl-1,4-dihydro-4-oxo-7-[4-(2-oxo-1,3-dioxol-4-yl-méthyl)-1-pipérazinyl]-3-quinoléine-carboxyliques selon la revendication 1, caractérisés en ce que

R représente un atome d'hydrogène,
$R^1$ représente un groupe méthyle,
$R^2$ et $R^3$ peuvent être identiques ou différents et représentent chacun un atome d'hydrogène, un groupe groupe méthyle, un groupe cyclohexyle, un groupe thiényle ou un groupe phényle éventuellement substitué une ou deux fois par un atome de fluor, un atome de chlore, un atome de brome, un groupe méthyle, un groupe hydroxyle, un groupe méthoxy, un groupe benzyloxy, un groupe amino, un groupe pipéridino ou un groupe nitro,
$X^1$ représente un atome de fluor, un atome de chlore ou un atome de brome, et
$X^2$ représente un atome d'hydrogène, un atome de fluor, un atome de chlore ou un atome de brome.

4. Acide 1-cyclopropyl-6-fluoro-1,4-dihydro-7-[4-(5-méthyl-2-oxo-1,3-dioxol-4-yl-méthyl)-1-pipérazinyl]-4-oxo-3-quinoléine-carboxylique,

acides 1-cyclopropyl-1,4-dihydro-4-oxo-7-[4-(2-oxo-1,3-dioxol-4-yl-méthyl)-1-pipérazinyl]-3-quinoléine-carboxyliques choisis parmi le groupe comprenant:

l'acide 1-cyclopropyl-6-fluoro-1,4-dihydro-4-oxo-7-[4-(2-oxo-1,3-dioxol-4-yl-méthyl)-1-pipérazinyl]-3-quinoléine-carboxylique,

l'acide 7-[4-(5-tert.-butyl-2-oxo-1,3-dioxol-4-yl-méthyl)-1-pipérazinyl]-1-cyclopropyl-6-fluoro-1,4-dihydro-4-oxo-3-quinoléine-carboxylique,

l'acide 1-cyclopropyl-6-fluoro-1,4-dihydro-4-oxo-7-[4-(2-oxo-5-phényl-1,3-dioxol-4-yl-méthyl)-1-pipérazinyl]-3-quinoléine-carboxylique,

l'acide 1-cyclopropyl-6-fluoro-1,4-dihydro-4-oxo-7-[4-(4,5,6,7-tétrahydro-2-oxo-1,3-benzdioxol-4-yl)-1-pipérazinyl]-3-quinoléine-carboxylique,

l'acide 1-cyclopropyl-6-fluoro-7-[3-(4-fluorophényl)-4-(5-méthyl-2-oxo-1,3-dioxol-4-yl-méthyl)-1-pipérazinyl]-1,4-dihydro-4-oxo-3-quinoléine-carboxylique,

l'acide 1-cyclopropyl-6-fluoro-1,4-dihydro-4-oxo-7-[4-(5-méthyl-2-oxo-1,3-dioxol-4-yl-méthyl)-3-(2-thiényl)-1-pipérazinyl]-3-quinoléine-carboxylique,

l'acide 1-cyclopropyl-6,8-difluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 1-cyclopropyl-6,8-difluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,

l'acide 1-cyclopropyl-6,8-difluoro-1,4-dihydro-7-[4-(5-méthyl-2-oxo-1,3-dioxol-4-yl-méthyl)-3-phényl-1-pipérazinyl]-4-oxo-3-quinoléine-carboxylique,

l'acide 1-cyclopropyl-6,8-difluoro-1,4-dihydro-7-[4-(5-méthyl-2-oxo-1,3-dioxol-4-yl-méthyl)-3-(2-thiényl)-1-pipérazinyl]-4-oxo-3-quinoléine-carboxylique,

l'acide 6-chloro-7-[3-cyclohexyl-4-(5-méthyl-2-oxo-1,3-dioxol-4-yl-méthyl)-1-pipérazinyl]-1-cyclopropyl-8-fluoro-1,4-dihydro-4-oxo-3-quinoléine-carboxylique,

l'acide 8-chloro-1-cyclopropyl-6-fluoro-1,4-dihydro-7-[4-(5-éthyl-2-oxo-1,3-dioxol-4-yl-méthyl)-1-pipérazinyl]-4-oxo-3-quinoléine-carboxylique,

l'acide 8-chloro-1-cyclopropyl-6-fluoro-1,4-dihydro-7-[3-méthyl-4-(5-méthyl-2-oxo-1,3-dioxol-4-yl-méthyl)-1-pipérazinyl]-4-oxo-3-quinoiéine-carboxylique,

l'acide 6,8-dichloro-1-cyclopropyl-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[4-(5-méthyl-2-oxo-1,3-dioxol-4-yl-méthyl)-3-phényl-1-pipérazinyl]-4-oxo-3-quinoléine-carboxylique,
l'acide 1-cyclopropyl-6-fluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,
l'acide 1-cyclopropyl-6-fluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,
l'acide 7-[3-(4-chlorophényl)-4-(5-méthyl-2-oxo-1,3-dioxol-4-yl-méthyl)-1-pipérazinyl]-1-cyclopropyl-6-fluoro-1,4-dihydro-4-oxo-3-quinoléine-carboxylique,
l'acide 7-[3-(4-bromophényl)-4-(5-méthyl-2-oxo-1,3-dioxol-4-yl-méthyl)-1-pipérazinyl]-1-cyclopropyl-6-fluoro-1,4-dihydro-4-oxo-3-quinoléine-carboxylique,
l'acide 1-cyclopropyl-6-fluoro-1,4-dihydro-7-[3-(4-méthoxyphényl)-4-(5-méthyl-2-oxo-1,3-dioxol-4-yl-méthyl)-1-pipérazinyl]-4-oxo-3-quinoléine-carboxylique,
l'acide1-cyclopropyl-6-fluoro-1,4-dihydro-7-[3-(4-hydroxyphényl)-4-(5-méthyl-2-oxo-1,3-dioxol-4-yl-méthyl)-1-pipérazinyl]-4-oxo-3-quinoléine-carboxylique,
l'acide 1-cyclopropyl-6-fluoro-1,4-dihydro-7-[4-(5-méthyl-2-oxo-1,3-dioxol-4-yl-méthyl)-3-(4-méthylphényl)-1-pipérazinyl]-4-oxo-3-quinoléine-carboxylique,
l'acide 1-cyclopropyl-6-fluoro-1,4-dihydro-7-[4-(5-méthyl-2-oxo-1,3-dioxol-4-yl-méthyl)-3-(4-pipéridino-phényl)-1-pipérazinyl]-4-oxo-3-quinoléine-carboxylique,
l'acide 1-cyclopropyl-6,8-difluoro-1,4-dihydro-7-[4-(5-méthyl-2-oxo-1,3-dioxol-4-yl-méthyl)-1-pipérazinyl]-4-oxo-3-quinoléine-carboxylique.

5. Procédé de préparation d'acides 1-cyclopropyl-1,4-dihydro-4-oxo-7-[4-(2-oxo-1,3-dioxol-4-yl-méthyl-1-pipérazinyl]-3-quinoléine-carboxylique de formule (I) selon la revendication 1

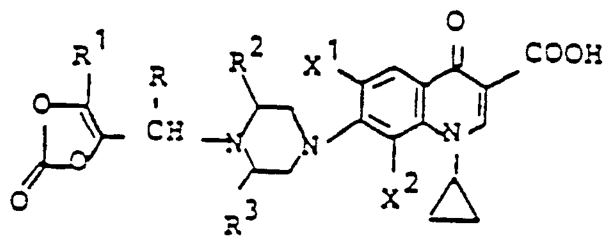

(I),

où

R, $R^1$, $R^2$, $R^3$, $X^1$ et $X^2$ ont les significations indiquées dans la revendication 1, caractérisé en ce qu'on fait réagir des acides 1-cyclopropyl-1,4-dihydro-4-oxo-7-(1-pipérazinyl)-3-quinoléine-carboxyliques de formule II:

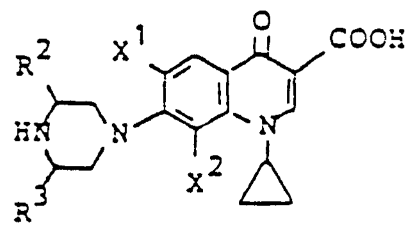

(II),

dans laquelle $R^2$, $R^3$, $X^1$ et $X^2$ ont les significations indiquées dans la revendication 1, avec des composés de formule (III):

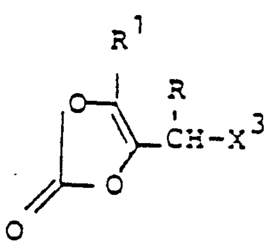

(III),

dans laquelle $R^1$ et R ont les significations indiquées dans la revendication 1, et

$X^3$ représente un atome de chlore, un atome de brome ou un atome d'iode ou encore un radical $O-SO_2-R^4$,

$R^4$ représentant un groupe méthyle, un groupe trifluorométhyle, un groupe phényle ou un groupe 4-méthylphényle

éventuellement en présence d'agents fixateurs d'acides.

6. Acides 1-cyclopropyl-1,4-dihydro-4-oxo-7-[4-(2-oxo-1,3-dioxol-4-yl-méthyl)-1-pipérazinyl]-3-quinoléine-carboxyliques de formule (I) selon la revendication 1

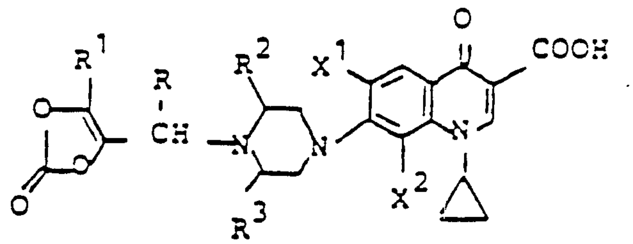

(I)

dans laquelle

R, $R^1$, $R^2$, $R^3$, $X^1$ et $X^2$ ont les significations indiquées dans la revendication 1,

en vue de les appliquer lors du traitement thérapeutique du corps humain ou animal.

7. Médicaments contenant des acides 1-cyclopropyl-1,4-dihydro-4-oxo-7-[4-(2-oxo-1,3-dioxol-4-yl-méthyl)-1-pipérazinyl ]-3-quinoléine-carboxyliques de formule (I) selon la revendication 1

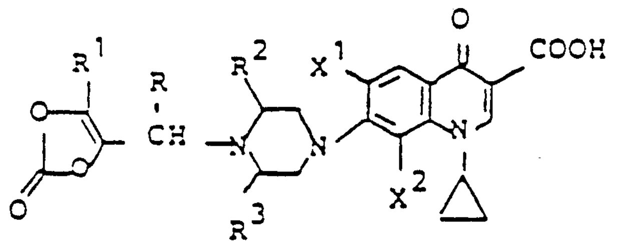

(I),

dans laquelle

R, $R^1$, $R^2$, $R^3$, $X^1$ et $X^2$ ont les significations indiquées dans la revendication 1,

ainsi que leurs sels de métaux alcalins et alcalino-terreux, leurs sels d'addition d'acide et leurs hydrates pharmaceutiquement utiles.

8. Utilisation d'acides 1-cyclopropyl-1,4-dihydro-4-oxo-7-[4-(2-oxo-1,3-dioxol-4-yl-méthyl)-1-pipérazinyl]-3-quinoléine-carboxyliques de formule (I) selon la revendication 1:

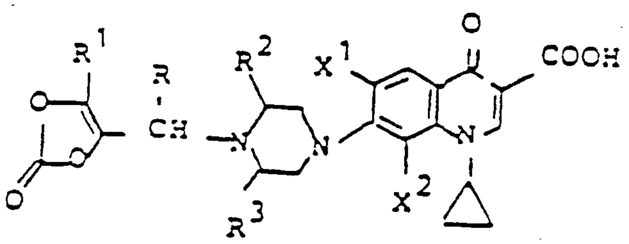

(I)

dans laquelle

R, $R^1$, $R^2$, $R^3$, $X^1$ et $X^2$ ont les significations indiquées dans la revendication 1,

ainsi que de leurs sels de métaux alcalins et alcalino-terreux, leurs sels d'addition d'acide et leurs hydrates pharmaceutiquement utiles pour la préparation de médicaments.

9. Utilisation des acides suivants:

l'acide 1-cyclopropyl-6-fluoro-1,4-dihydro-7-[4-(5-méthyl-2-oxo-1,3-dioxol-4-yl-méthyl)-1-pipérazinyl]-4-oxo-3-quinoléine-carboxylique,

l'acide 1-cyclopropyl-6-fluoro-1,4-dihydro-4-oxo-7-[4-(2-oxo-1,3-dioxol-4-yl-méthyl)-1-pipérazinyl]-3-quinoléine-carboxylique,

l'acide 7-[-4-(5-tert.-butyl-2-oxo-1,3-dioxol-4-yl-méthyl)-1-pipérazinyl]-1-cyclopropyl-6-fluoro-1,4-dihydro-4-oxo-3-quinoléine-carboxylique,

l'acide 1-cyclopropyl-6-fluoro-1,4-dihydro-4-oxo-7-[4-(2-oxo-5-phényl-1,3-dioxol-4-yl-méthyl)-1-pipérazinyl]-3-quinoléine-carboxylique,

l'acide 1-cyclopropyl-6-fluoro-1,4-dihydro-4-oxo-7-[4-(4,5,6,7-tétrahydro-2-oxo-1,3-benzdioxol-4-yl)-1-pipérazinyl]-3-quinoléine-carboxylique,

l'acide 1-cyclopropyl-6-fluoro-7-[3-(4-fluorophényl)-4-(5-méthyl-2-oxo-1,3-dioxol-4-yl-méthyl)-1-pipérazinyl]-1,4-dihydro-4-oxo-3-quinoléine-carboxylique,

l'acide 1-cyclopropyl-6-fluoro-1,4-dihydro-4-oxo-7-[4-(5-méthyl-2-oxo-1,3-dioxol-4-yl-méthyl)-3-(2-thiényl)-1-pipérazinyl]-3-quinoléine-carboxylique,

l'acide 1-cyclopropyl-6,8-difluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 1-cyclopropyl-6,8-difluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,

l'acide 1-cyclopropyl-6,8-difluoro-1,4-dihydro-7-[4-(5-méthyl-2-oxo-1,3-dioxol-4-yl-méthyl)-3-phényl-1-pipérazinyl]-4-oxo-3-quinoléine-carboxylique,

l'acide 1-cyclopropyl-6,8-difluoro-1,4-dihydro-7-[4-(5-méthyl-2-oxo-1,3-dioxol-4-yl-méthyl)-3-(2-thiényl)-1-pipérazinyl]-4-oxo-3-quinoléine-carboxylique,

l'acide 6-chloro-7-[3-cyclohexyl-4-(5-méthyl-2-oxo-1,3-dioxol-4-yl-méthyl)-1-pipérazinyl]-1-cyclopropyl-8-fluoro-1,4-dihydro-4-oxo-3-quinoléine-carboxylique,

l'acide 8-chloro-1-cyclopropyl-6-fluoro-1,4-dihydro-7-[4-(5-méthyl-2-oxo-1,3-dioxol-4-yl-méthyl)-1-pipérazinyl]-4-oxo-3-quinoléine-carboxylique,

l'acide 8-chloro-1-cyclopropyl-6-fluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 6,8-dichloro-1-cyclopropyl-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[3-méthyl-4-(5-méthyl-2-oxo-1,3-dioxol-4-yl-méthyl-1-pipérazinyl]-4-oxo-3-quinoléine-carboxylique,

l'acide 6-chloro-1-cyclopropyl-8-fluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,

l'acide 6-chloro-1-cyclopropyl-8-fluoro-1,4-dihydro-7-[4-(5-méthyl-2-oxo-1,3-dioxol-4-yl-méthyl)-3-phényl-1-pipérazinyl]-4-oxo-3-quinoléine-carboxylique,

l'acide 1-cyclopropyl-6-fluoro-1,4-dihydro-7-[3-méthyl-4-(5-méthyl-2-oxo-1,3-dioxol-4-yl-méthyl)-1-pipérazinyl]-4-oxo-3-quinoléine-carboxylique,

l'acide 1-cyclopropyl-6-fluoro-1,4-dihydro-7-[3,5-diméthyl-4-(5-méthyl-2-oxo-1,3-dioxol-4-yl-méthyl)-1-pipérazinyl]-4-oxo-3-quinoléine-carboxylique,

l'acide 7-[3-(4-chlorophényl)-4-(5-méthyl-2-oxo-1,3-dioxol-4-yl-méthyl)-1-pipérazinyl]-1-cyclopropyl-6-fluoro-1,4-dihydro-4-oxo-3-quinoléine-carboxylique,

l'acide 7-[3-(4-bromophényl)-4-(5-méthyl-2-oxo-1,3-dioxol-4-yl-méthyl)-1-pipérazinyl]-1-cyclopropyl-6-fluoro-1,4-dihydro-4-oxo-3-quinoléine-carboxylique,

l'acide 1-cyclopropyl-6-fluoro-1,4-dihydro-7-[3-(4-méthoxyphényl)-4-(5-méthyl-2-oxo-1,3-dioxol-4-yl-méthyl)-1-pipérazinyl]-4-oxo-3-quinoléine-carboxylique,

l'acide 1-cyclopropyl-6-fluoro-1,4-dihydro-7-[3-(4-hydroxyphényl)-4-(5-méthyl-2-oxo-1,3-dioxol-4-yl-méthyl)-1-pipérazinyl]-4-oxo-3-quinoléine-carboxylique,

l'acide 1-ncyclopropyl-6-fluoro-1,4-dihydro-7-[4-(5-méthyl-2-oxo-1,3-dioxol-4-yl-méthyl)-3-(4-méthylphényl)-1-pipérazinyl]-4-oxo-3-quinoléine-carboxylique,

l'acide 1-cyclopropyl-6-fluoro-1,4-dihydro-7-[4-(5-méthyl-2-oxo-1,3-dioxol-4-yl-méthyl)-3-(4-pipéridino-phényl)-1-pipérazinyl]-4-oxo-3-quinoléine-carboxylique,

l'acide 1-cyclopropyl-6,8-difluoro-1,4-dihydro-7-[4-(5-méthyl-2-oxo-1,3-dioxol-4-yl-méthyl)-1-pipérazinyl]-4-oxo-3-quinoléine-carboxylique,

pour la préparation de médicaments.

10. Acides 1-cyclopropyl-1,4-dihydro-4-oxo-7-[4-(2-oxo-1,3-dioxol-4-yl-méthyl)-1-pipérazinyl]-3-quinoléine-carboxyliques choisis parmi le groupe comprenant:

l'acide 1-cyclopropyl-6-fluoro-1,4-dihydro-7-[4-(5-méthyl-2-oxo-1,3-dioxol-4-yl-méthyl)-1-pipérazinyl]-4-oxo-3-quinoléine-carboxylique;

l'acide 8-chloro-1-cyclopropyl-6-fluoro-1,4-dihydro-7-[4-(5-méthyl-2-oxo-1,3-dioxol-4-yl-méthyl)-1-pipérazinyl]-4-oxo-3-quinoléine-carboxylique;

l'acide 8-chloro-1-cyclopropyl-6-fluoro-1,4-dihydro-7-[3-méthyl-4-(5-méthyl-2-oxo-1,3-dioxol-4-yl-méthyl)-1-pipérazinyl]-4-oxo-3-quinoléine-carboxylique;

l'acide 1-cyclopropyl-6,8-difluoro-1,4-dihydro-7-[4-(5-méthyl-2-oxo-1,3-dioxol-4-yl-méthyl)-1-pipérazinyl]-4-oxo-3-quinoléine-carboxylique, et

l'acide 1-cyclopropyl-6,8-difluoro-1,4-dihydro-7-[3-méthyl-4-(5-méthyl-2-oxo-1,3-dioxol-4-yl-méthyl)-1-pipérazinyl]-4-oxo-3-quinoléine-carboxylique.